# EUROPEAN PATENT APPLICATION

(11) **EP 4 278 888 A1**
(43) Date of publication of application: **22.11.2023**
(21) Application number: 21919646.6
(22) Date of filing: 13.12.2021
(51) Int. Cl.: A01G 7/00

(54) **MOISTURE SENSOR CONTROL DEVICE, MOISTURE SENSOR CONTROL SYSTEM, AND MOISTURE SENSOR CONTROL METHOD**

(30) Priority: 14.01.2021 JP 2021004211
(71) Applicant: Sony Group Corporation, Tokyo 108-0075 (JP)
(72) Inventor: KOBAYASHI, Takashi, Tokyo 108-0075 (JP)
(74) Representative: D Young & Co LLP
(86) International application number: PCT/JP2021/045792
(87) International publication number: WO 2022/153757

(57) **Abstract**

[Object] To provide a moisture sensor controlling apparatus, a moisture sensor controlling system, and a moisture sensor controlling method that make it possible to reduce power consumption more effectively.

[Solving Means] A moisture sensor controlling apparatus according to an embodiment of the present technology includes a sensor state selecting section. The sensor state selecting section controls an operation state of at least one moisture sensor on the basis of environment information regarding an environment of soil in which crops are planted, and on the basis of position information regarding a position of the at least one moisture sensor, the at least one moisture sensor being installed in the soil and detecting a moisture amount in the soil.

## Description

### Technical Field

The present technology relates to a moisture sensor controlling apparatus that controls a moisture sensor that detects moisture in soil, a moisture sensor controlling system, and a moisture sensor controlling method.

### Background Art

In the field of agriculture, there is a need to spray or pour water with appropriate frequency or at an appropriate timing according to the moisture content in soil, in order to healthily grow crops. For example, Patent Literature 1 discloses detecting the moisture content in soil and the rate of change in the moisture content in soil, and controlling a timing of operating each moisture sensor on the basis of a determined threshold for the moisture content in soil.

In order to detect the moisture content in soil in a large region with a high degree of accuracy, there is a need to install a large number of moisture sensors, and there is a need for a method for supplying power sufficient to operate the large number of moisture sensors for a long time.

### Citation List

### Patent Literature

Patent Literature 1: United States Patent Application Publication No. 2010/147389

### Disclosure of Invention

### Technical Problem

For example, it costs a lot to supply power to a large number of moisture sensors outdoors, where there is a need for, for example, large-capacity batteries and solar power systems with high power output. Thus, there is a demand for a reduction in power consumption upon detecting the moisture content in soil, in order to reduce costs.

In view of the circumstances described above, it is an object of the present technology to provide a moisture sensor controlling apparatus, a moisture sensor controlling system, and a moisture sensor controlling method that make it possible to reduce power consumption more effectively.

### Solution to Problem

In order to achieve the object described above, a moisture sensor controlling apparatus according to an embodiment of the present technology includes a sensor state selecting section.

The sensor state selecting section controls an operation state of at least one moisture sensor on the basis of environment information regarding an environment of soil in which crops are planted, and on the basis of position information regarding a position of the at least one moisture sensor, the at least one moisture sensor being installed in the soil and detecting a moisture amount in the soil.

This configuration makes it possible to control an operation state of the moisture sensor, such as only operating the moisture sensor estimated to have to, for example, be detected, on the basis of a relationship between environment information regarding an environment of soil, and on the basis of position information regarding a position of the moisture sensor. This makes it possible to reduce power consumption.

As the controlling the operation state of the moisture sensor, the sensor state selecting section may control at least one of whether to supply power to the moisture sensor in order to detect the moisture amount in the soil, or how frequent the moisture amount in the soil is to be detected by the moisture sensor.

The moisture sensor may include a communication section that transmits detection data of the detection performed by the moisture sensor, and
as the controlling the operation state of the moisture sensor, the sensor state selecting section may control at least one of whether the detection data of the detection performed by the moisture sensor is to be transmitted by the communication section, or how frequent the detection data of the detection performed by the moisture sensor is to be transmitted.

The moisture sensor may detect relative permittivity of the soil using an electromagnetic wave in order to detect the moisture amount in the soil, and
as the controlling the operation state of the moisture sensor, the sensor state selecting section may control a band of the electromagnetic wave.

The environment information may include at least one of position information regarding a position of a portion of the crop, or water supply information regarding water supply to the soil.

The moisture sensor may further include an environment data processor that calculates the environment information using environment data.

The environment data processor may calculate the position information regarding a position of a portion of the crop on the basis of image data that is the environment data and is acquired by an image-capturing section that captures an image of the crop above the ground, and
the sensor state selecting section may control the operation state of each moisture sensor using the position information regarding a position of a portion of the crop on the basis of a distance between the portion of the crop and the moisture sensor.

The environment data processor may calculate, from the image data of the crop, the position information regarding a position of the portion of the crop, the portion of the crop being situated above the ground.

The environment data processor may refer to growth information regarding growth of the crop that is acquired in advance, and may calculate, from the image data of the crop, the position information regarding a position of the portion of the crop, the portion of the crop being situated in the soil.

The moisture sensors of a plurality of the moisture sensors may be intermittently arranged in at least one of a horizontal direction of the soil or a depth direction of the soil, and
the sensor state selecting section may control the operation state of each moisture sensor on the basis of a distance between the position of the portion of the crop and the moisture sensor, the position of the portion of the crop being estimated by the environment data processor, the portion of the crop being situated in the soil.

The environment data processor may calculate the water supply information regarding water supply to the soil, using at least one of water supply data of water supply to the soil that is performed by a water spraying apparatus, rainfall data of rainfall in the soil, or detection data of the detection performed by the moisture sensor installed in the soil, the water supply data, the rainfall data, and the detection data being the pieces of environment data.

The water supply information regarding water supply to the soil may include soil-water-permeation-region information regarding a soil-water-permeation region in the soil, and
the environment data processor may calculate the soil-water-permeation-region information using at least one of water supply data of water supply to the soil that is performed by a water spraying apparatus, rainfall data of rainfall in the soil, or detection data of the detection performed by the moisture sensor installed in the soil, and using at least one of soil classification for the soil or a speed at which soil water moves in the soil, the water supply data, the rainfall data, the detection data, the soil classification, and the soil water moving speed being the pieces of environment data.

The sensor state selecting section may control the operation states of the respective moisture sensors of a plurality of the moisture sensors.

In order to achieve the object described above, a moisture sensor controlling system according to an embodiment of the present technology includes
at least one moisture sensor that is installed in soil in which crops are planted, the at least one moisture sensor detecting a moisture amount in the soil; and a sensor state selecting section that controls an operation state of the at least one moisture sensor on the basis of environment information regarding an environment of the soil, and on the basis of position information regarding a position of the at least one moisture sensor.

In order to achieve the object described above, a moisture sensor controlling method according to an embodiment of the present technology includes controlling an operation state of at least one moisture sensor on the basis of environment information regarding an environment of soil in which crops are planted, and on the basis of position information regarding a position of the at least one moisture sensor, the at least one moisture sensor being installed in the soil and detecting a moisture amount in the soil.

### Brief Description of Drawings

[Fig. 1] Fig. 1 schematically illustrates a schematic configuration of a moisture sensor controlling system according to embodiments of the present technology, and schematically illustrates how a moisture sensor is installed in soil.
[Fig. 2] Fig. 2 is a block diagram of a functional configuration of the moisture sensor controlling system.
[Fig. 3] Fig. 3 schematically illustrates how the moisture sensor is installed.
[Fig. 4] Fig. 4 schematically illustrates a schematic configuration of a moisture amount measuring apparatus.
[Fig. 5] Fig. 5 is a flowchart used to describe a moisture sensor controlling method according to a first embodiment of the present technology.
[Fig. 6] Fig. 6 is a flowchart used to describe the moisture sensor controlling method according to a second embodiment of the present technology.
[Fig. 7] Fig. 7 is a flowchart used to describe the moisture sensor controlling method according to a third embodiment of the present technology.
[Fig. 8] Fig. 8 is a flowchart used to describe the moisture sensor controlling method according to a fourth embodiment of the present technology.
[Fig. 9] Fig. 9 is a flowchart used to describe a moisture sensor controlling method according to a fifth embodiment of the present technology.
[Fig. 10] Fig. 10 is a flowchart used to describe the moisture sensor controlling method according to a sixth embodiment of the present technology.
[Fig. 11] Fig. 11 is a flowchart used to describe the moisture sensor controlling method according to a seventh embodiment of the present technology.
[Fig. 12] Fig. 12 is a flowchart used to describe the moisture sensor controlling method according to an eighth embodiment of the present technology.

### Mode(s) for Carrying Out the Invention

Embodiments according to the present technology will now be described below with reference to the drawings.

### <Schematic Configuration of Moisture Sensor Controlling System>

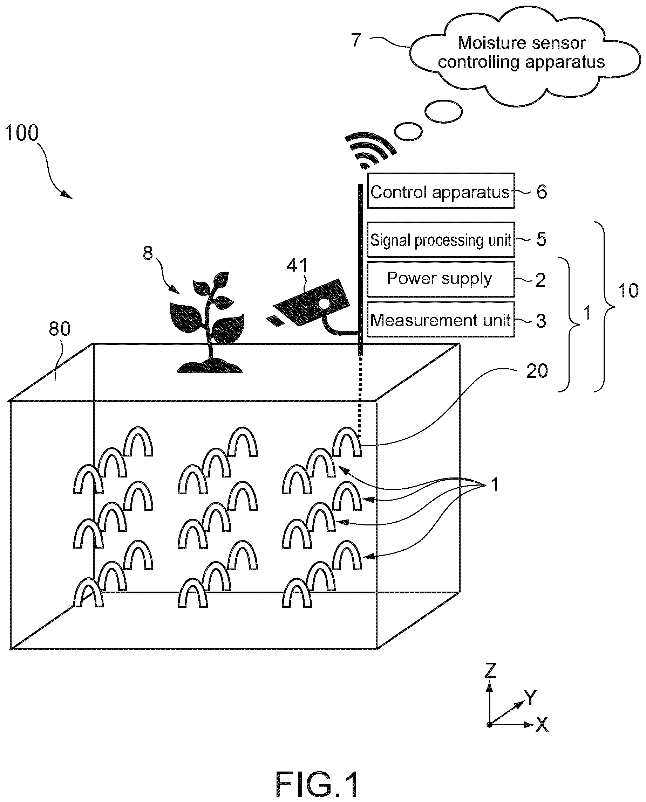

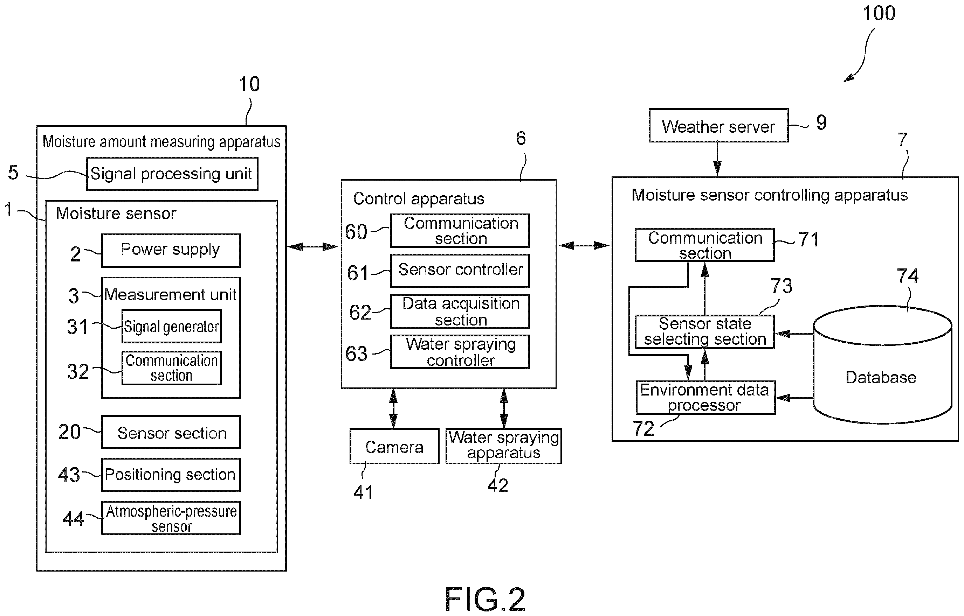

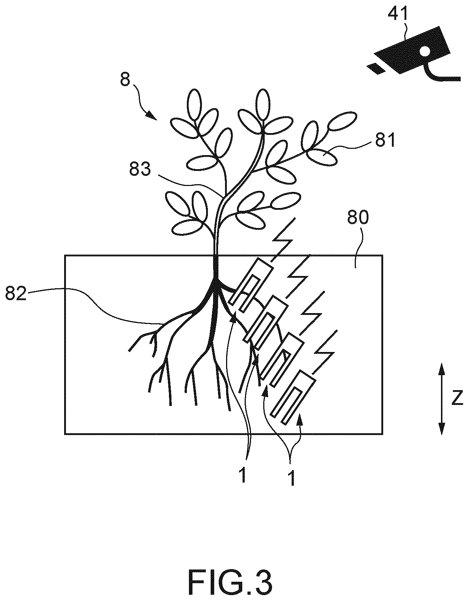

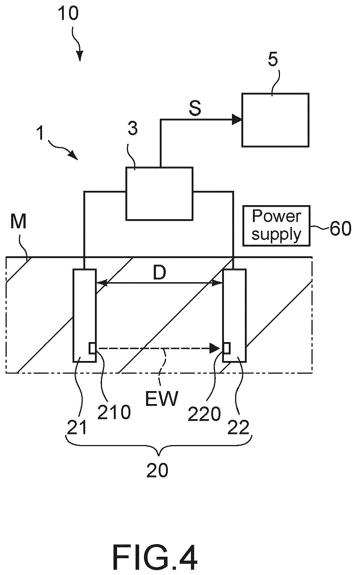

Fig. 1 illustrates a schematic configuration of a moisture sensor controlling system according to an embodiment of the present technology, and schematically illustrates how a moisture sensor is installed in soil. Fig. 2 is a block diagram of a functional configuration of the moisture sensor controlling system. Fig. 3 schematically illustrates how the moisture sensor is installed. Fig. 4 schematically illustrates a schematic configuration of a moisture amount measuring apparatus.

In Fig. 1, an XY plane formed by an X axis and a Y axis that are orthogonal to each other corresponds to a horizontal direction. A Z axis that extends in a vertical direction that is orthogonal to the XY plane corresponds to a depth direction of soil.

As illustrated in Figs. 1 and 2, a moisture sensor controlling system 100 includes a moisture amount measuring apparatus 10, a control apparatus 6, a moisture sensor controlling apparatus 7, a camera 41, and a water spraying apparatus 42.

In the present embodiment, an example of applying the present technology to control of an operation state of a moisture sensor 1 that is a portion of the moisture amount measuring apparatus 10 used to measure a moisture amount in soil 80 where crops 8 will grow, is described. The moisture sensor 1 is installed in the soil 80.

The crops 8 refer to general plants cultivated in the fields. Examples of the crops 8 include food crops primarily eaten as staple diets, vegetables, fruit trees, garden products such as ornamental flowering plants, forage crops used as fertilizers for the livestock, and green manure crops used as crop fertilizers.

Examples of the "soil" include not only natural soil but also artificial soil.

In the moisture sensor controlling system 100, the moisture sensor controlling apparatus 7 controls an operation state of the moisture sensor 1 on the basis of environment information regarding an environment of the soil 80 and position information regarding a position of the moisture sensor 1 in the soil 80. More specifically, the control apparatus 6 controls the moisture sensor 1 on the basis of an operation state of the moisture sensor 1 that is selected by the moisture sensor controlling apparatus 7.

The environment information regarding an environment of the soil 80 includes position information regarding a position of the crop 8 planted in the soil 80, and water supply information regarding water supply to the soil 80. In the moisture sensor controlling system 100, the moisture sensor controlling apparatus 7 controls an operation state of the moisture sensor 1 on the basis of at least one of the position information regarding a position of the crop 8 or the water supply information regarding water supply to the soil 80, and on the basis of the position information regarding a position of the moisture sensor 1.

The position information regarding a position of the crop 8 is generated using, for example, image data of the crop 8 that is environment data and is acquired by the camera 41.

The water supply information regarding water supply to the soil 80 is generated using, for example, at least one of water-spraying data of water spraying performed by the water spraying apparatus 42, rainfall data acquired from a weather server 9 that provides weather information services, or data of a moisture amount in soil that is calculated by the moisture amount measuring apparatus 10, where the water-spraying data, the rainfall data, and the data of a moisture amount in soil are pieces of environment data.

The position information regarding a position of the moisture sensor 1 in the soil 80 is information that uniquely indicates a position of the moisture sensor 1 in the soil 80.

For example, the position information regarding a position of the moisture sensor 1 is measured in advance when the moisture sensor is installed in the soil 80, and is stored in a database 74 described later. Alternatively, the position information regarding a position of the moisture sensor 1 may be position information that is generated by a known technology such as a positioning section 43 or an atmospheric-pressure sensor 44 and recorded automatically after the moisture sensor is installed.

An example in which the position information regarding a position of the moisture sensor 1 is stored in the database 74 of the moisture sensor controlling apparatus 7 is described in the present embodiment.

This is described in detail below.

### <Camera>

The camera 41 serving as an image-capturing section acquires image data of the crop 8 as environment data.

As illustrated in Figs. 1 and 3, the camera 41 is installed above the ground. The camera 41 acquires image data of a portion of the crop 8 planted in the soil 80, the portion being situated above the ground. The number of cameras 41 installed, and a location to install the camera 41 may be set as appropriate according to the soil area and the kind of the crop 8.

The image data acquired by the camera 41 is acquired by a data acquisition section 62 that is included in the control apparatus 6 and will be described later, and is transmitted to the moisture sensor controlling apparatus 7.

### <Water Spraying Apparatus>

The water spraying apparatus 42 is, for example, a sprinkler or a water spraying hose, and water is supplied to the soil 80 manually or automatically using the water spraying apparatus 42.

Data of water spraying and pouring on the soil 80 that is performed by the water spraying apparatus 42, that is, water supply data of water supply to the soil 80 that is performed by the water spraying apparatus 42 is environment data.

The water supply data of water supply to the soil 80 that is performed by the water spraying apparatus 42 includes a time of starting spraying water, a time of finishing spraying water, and a water spraying area. For example, when a plurality of water spraying apparatuses 42 is installed in the soil 80 having a large area, setting may be performed such that the respective water spraying apparatuses spray water at different timings. In this case, the water spraying area in soil differs depending on a period of time. Further, even if sprinklers or water spraying hoses spray water over the entirety of soil at the same water spraying timing, there is a possibility that water will not be evenly sprayed over the entirety of target soil depending on, for example, how the sprinklers or the water spraying hoses are installed. This may result in there unevenly existing water spraying areas. Information regarding a water spraying area makes it possible to understand over which area in target soil water is being sprayed. A water spraying area in soil can be estimated using installation position information regarding a position at which the water spraying apparatus is installed or using the kind of water spraying apparatus.

The water supply data of water supply to the soil 80 that is performed by the water spraying apparatus 42 is acquired by the data acquisition section 62 that is included in the control apparatus 6 and will be described later, and is transmitted to the moisture sensor controlling apparatus 7.

### <Moisture Amount Measuring Apparatus>

As illustrated in Figs. 1, 2, and 4, the moisture amount measuring apparatus 10 includes the moisture sensor 1 and a signal processing unit 5.

Ordinarily used apparatuses may be used as the moisture amount measuring apparatus 10 with no particular restriction. For example, an apparatus used to measure the relative permittivity of soil using time domain reflectometry (TDR), frequency domain reflectometry (FDR), or amplitude domain reflectometry (ADR) may be used, and a moisture amount in soil may be calculated from the measured relative permittivity.

The moisture sensor 1 acquires characteristics of a propagation of an electromagnetic wave in the soil 80, and generates a measurement signal S (which may be hereinafter referred to as detection data) used to calculate relative permittivity of the soil 80. A configuration of the moisture sensor will be described in detail later.

The signal processing unit 5 includes an information processing apparatus that receives the measurement signal S from the moisture sensor 1 and calculates a moisture amount in the soil 80 on the basis of the measurement signal S. For example, the Topp's formula is used to calculate the moisture amount, and a proportion of moisture content by volume [%] in the soil 80 is calculated as the moisture amount. The signal processing unit 5 may include, for example, a communication section and a display section. The communication section is capable of communicating with a communication section 32 that is a portion of the moisture sensor 1 and with a communication section 60 of the control apparatus 6. The communication section 32 and the communication section 60 will be described later. The display section is capable of displaying thereon, for example, information regarding, for example, relative permittivity of soil and a moisture amount in the soil. The moisture amount in soil being calculated by the signal processing unit 5 may be transmitted to the control apparatus 6 as environment data.

### [Configuration of Moisture Sensor]

At least one moisture sensor 1 is arranged in the soil 80. Here, an example of arranging a plurality of moisture sensors 1 is described. In the present embodiment, moisture sensors 1 of a plurality of moisture sensors 1 are intermittently arranged in the horizontal direction and in the depth direction (a vertical direction Z). Spacing with which a plurality of moisture sensors 1 is installed can be set as appropriate according to an expected crop 8, and, for example, the moisture sensors 1 of the plurality of moisture sensors 1 are installed with spacing of from about 3 m to about 7 m in the horizontal direction and with spacing of from about 10 cm to about 20 cm in the depth direction.

As illustrated in Fig. 2, the moisture sensor 1 includes a power supply 2, a measurement unit 3, a sensor section 20, the positioning section 43, and the atmospheric-pressure sensor 44. Each structural element is described below.

### (Sensor Section)

As illustrated in Fig. 4, the sensor section 20 includes a transmission probe 21 and a reception probe 22. The sensor section 20 includes antenna sections 210 and 220 that are arranged in the soil 80 and enable transmission and reception of an electromagnetic wave EW of a specified frequency between the transmission probe 21 and the reception probe 22.

The transmission probe 21 and the reception probe 22 are embedded in the soil 80 in a generally vertical pose such that they are situated at a distance D from each other to face each other.

The transmission probe 21 is connected to an output terminal of the measurement unit 3, and transmits a transmission signal from the measurement unit 3 to the antenna section 210. The antenna section 210 is provided on a tip (an end) of the transmission probe 21 or near the tip of the transmission probe 21, and transmits the electromagnetic wave EW corresponding to the transmission signal to the reception probe 22.

The reception probe 22 is connected to an input terminal of the measurement unit 3, receives the electromagnetic wave EW using the antenna section 220, and inputs a reception signal to the control apparatus 6. The antenna section 220 is provided on a tip (an end) of the reception probe 22 or near the tip of the reception probe 22 such that the antenna section 220 faces the antenna section 210 of the transmission probe 21.

The antenna sections 210 and 220 are used to locally transmit and receive the electromagnetic wave EW at respective specified locations in the probes 21 and 22, and, typically, the antenna sections 210 and 220 are tiny antenna sections that are formed to be sufficiently small in size to not resonate the respective probes 21 and 22. This makes it possible to prevent the measurement accuracy from being decreased due to resonances of the probes 21 and 22.

### (Power Supply)

The power supply 2 is a drive source of the moisture sensor 1. For example, a battery may be used as the power supply 2, but the power supply 2 is not limited thereto. The power supply 2 supplies power to a signal generator 31 and the communication section 32 of the moisture sensor 1.

### (Measurement Unit)

The measurement unit 3 includes the signal generator 31 and the communication section 32. Typically, the measurement unit includes a network analyzer.

The signal generator 31 generates a measurement signal S that includes information regarding the characteristics of a propagation of the electromagnetic wave EW in the soil 80 between the antenna section 210 of the transmission probe 21 and the antenna section 220 of the reception probe 22.

The signal generator 31 includes, for example, a controller, a signal creating section, a phase shifter, and a mixer.

The controller controls respective structural elements of the measurement unit, where examples of the structural element include the signal creating section and the communication section 32.

The signal creating section creates a transmission signal of a specified frequency, and inputs the transmission signal to the transmission probe 21 through an amplifier and the output terminal. The signal creating section creates a pulse wave (a pulse signal) as the transmission signal, but may be configured to create a continuous wave as the transmission signal.

The phase shifter splits the transmission signal into two signals that are 90 degrees out of phase, and inputs the two signals to the mixer. The mixer mixes a reception signal with the two signals output from the phase shifter to modulate those signals into two response signals, the reception signal being input from the reception probe 22 through the input terminal and an amplifier, the two response signals being in quadrature to each other. These response signals are converted into a digital signal from an analog signal through an AD converter, and the measurement signal S is generated by the controller from the response signals.

The communication section 32 includes a communication module that includes, for example, an antenna for communication. The communication section 32 is used to wirelessly transmit the measurement signal S from the moisture sensor 1 to the signal processing unit 5. This makes it possible to provide the measurement signal S to the signal processing unit 5 arranged at a location different from an observation point. Without being limited thereto, the moisture sensor 1 may be connected to the signal processing unit 5 through, for example, a distribution cable.

### (Positioning Section and Atmospheric-Pressure Sensor)

The positioning section 43 is provided near each moisture sensor 1. The positioning section 43 receives, for example, a global navigation satellite system (GNSS) signal from a GNSS satellite (such as a Global Positioning System (GPS) signal from a GPS satellite) to perform positioning, and generates position data of a position of the moisture sensor 1, the position data including latitude and longitude.

The atmospheric-pressure sensor 44 is provided near each moisture sensor 1. Depth data that indicates position data of a position of the moisture sensor 1 in soil in the depth direction is generated on the basis of detection information regarding detection performed by the atmospheric-pressure sensor 44.

Position information regarding a position of the moisture sensor 1 can be calculated on the basis of the position data of a position detected by the positioning section 43 and on the basis of the position data of a position detected by the atmospheric-pressure sensor 44. The position data of a position detected by the positioning section 43 and the position data of a position detected by the atmospheric-pressure sensor 44 may be acquired by the data acquisition section 62 that is included in the control apparatus 6 and will be described later, and may be transmitted to the moisture sensor controlling apparatus 7. The calculated position information regarding a position of the moisture sensor 1 may be stored in the database 74 described later.

### <Control Apparatus>

The control apparatus 6 collectively controls a plurality of moisture sensors 1 situated in the soil 80. Further, the control apparatus 6 may control the water spraying apparatus 42, the camera 41, the positioning section 43, and the atmospheric-pressure sensor 44.

The control apparatus 6 includes, for example, the communication section 60, a sensor controller 61, the data acquisition section 62, and a water spraying controller 63.

### [Description of Each Structural Element of Control Apparatus]

### (Communication Section)

The communication section 60 includes, for example, a satellite communication module and a short-range wireless module.

The communication section 60 wirelessly transmits environment data acquired by the data acquisition section 62 to the moisture sensor controlling apparatus 7.

The environment data includes at least one of image data acquired by the camera 41, pieces of position data acquired by the positioning section 43 and the atmospheric-pressure sensor 44, water supply data of water supply performed by the water spraying apparatus 42, data of a moisture amount in soil that is calculated by the moisture amount measuring apparatus 10, or detection data detected by the moisture sensor 1 (the measurement signal S).

The communication section 60 wirelessly receives sensor state information regarding a state of the moisture sensor 1 that is calculated by the moisture sensor controlling apparatus 7 described later. The received sensor state information is output to the sensor controller 61.

The communication section 60 may wirelessly receive a signal for controlling spraying or pouring of water that is used to control the water spraying apparatus 42. For example, the signal for controlling spraying or pouring of water may be generated by the moisture sensor controlling apparatus 7, or may be generated by another information processing apparatus. The received signal for controlling spraying or pouring of water is output to the water spraying controller 63.

### (Sensor Controller)

The sensor controller 61 controls an operation state of the moisture sensor 1 on the basis of sensor state information received from the moisture sensor controlling apparatus 7 through the communication section 60. The sensor controller 61 individually controls moisture sensors 1 of a plurality of moisture sensors 1. The operation state of the moisture sensor 1 will be described later.

Further, the sensor controller 61 may control, for example, a timing of and the frequency in detection performed by a sensor. Examples of the sensor include the camera 41, the positioning section 43, and the atmospheric-pressure sensor 44.

### (Data Acquisition Section)

The data acquisition section 62 acquires image data from the camera 41, acquires pieces of position data of a position of the moisture sensor 1 from the positioning section 43 and the atmospheric-pressure sensor 44, acquires data of a moisture amount in soil from the moisture amount measuring apparatus 10, and acquires water supply data from the water spraying apparatus 42. The image data, the pieces of position data, the data of a moisture amount in soil, and the water supply data are acquired as environment data through the communication section 60. The acquired environment data is transmitted to the moisture sensor controlling apparatus 7 through the communication section 60.

### (Water Spraying Controller)

The water spraying controller 63 controls, for example, a timing of and the frequency in spraying or pouring of water that is performed by the water spraying apparatus 42, on the basis of a signal for controlling spraying or pouring of water that is received through the communication section 60.

### [Operation State of Moisture Sensor]

The operation state of the moisture sensor 1 includes a state A and a state B.

The state A is a state in which power is supplied from the power supply 2 to the signal generator 31 and the communication section 32 of the moisture sensor 1, detection is performed by the moisture sensor 1, and detection data of the detection performed by the moisture sensor 1 is transmitted to the signal processing unit 5. During the detection, the detection data (the measurement signal S) may be transmitted to the signal processing unit 5 as necessary or at specified intervals. It can be said that the state A is a state in which communication and computation processing is performed. The state A may be hereinafter referred to as a detection state.

The state B is a state in which no power is supplied by the power supply 2 and no detection is performed. In the state B, detection data of detection performed by the moisture sensor 1 is not transmitted to the signal processing unit 5. It can be said that the state B is a state in which communication and computation processing is not performed. The state B may be hereinafter referred to as a detection stop state.

Further, the state A may include a state A1 in which detection is performed frequently, and a state A2 in which detection is performed less frequently. Specifically, in the present embodiment, the frequency in detection is frequency with which a series of detection processing is performed, the series of detection processing including transmitting a transmission signal from the measurement unit 3 to the antenna section 210, receiving, using the antenna section 220, an electromagnetic wave EW corresponding to the transmission signal, and inputting a reception signal to the measurement unit 3. In the following description, the state A1 may be referred to as a frequent-detection state, and the state A2 may be referred to as a less-frequent-detection state.

Further, the operation state of the moisture sensor 1 includes a state C and a state D.

The state C is a state in which power is supplied from the power supply 2 to the signal generator 31 and the communication section 32 of the moisture sensor 1, detection is performed by the moisture sensor 1, and detection data of the detection performed by the moisture sensor 1 is transmitted to the signal processing unit 5. During the detection, the detection data may be transmitted to the signal processing unit 5 as necessary or at specified intervals. It can be said that the state C is a state in which communication and computation processing is performed.

The state D is a state in which power is supplied from the power supply 2 to the signal generator 31 of the moisture sensor and detection processing is performed, but no transmission power is supplied to the communication section 32 and processing of transmitting, to the signal processing unit 5, detection data of detection performed by the moisture sensor 1 is not performed.

In other words, the state C is a state in which transmission power is supplied to the communication section 32, whereas the state D is a state in which no transmission power is supplied to the communication section 32. In the following description, the state C may be referred to as a communication state, and the state D may be referred to as a communication stop state.

Further, the state C may include a state C1 in which detection data is transmitted frequently, and a state C2 in which detection data is transmitted less frequently. In the following description, the state C1 may be referred to as a frequent-communication state, and the state C2 may be referred to as a less-frequent-communication state.

### <Moisture Sensor Controlling Apparatus>

The moisture sensor controlling apparatus 7 performs a series of processing of selecting an operation state of the moisture sensor 1, on the basis of environment information regarding an environment of the soil 80 that is calculated using environment data, and on the basis of position information regarding a position of the moisture sensor 1 in the soil.

As described above, the environment information regarding an environment of the soil 80 includes position information regarding a position of the crop 8 and water supply information regarding water supply to the soil 80. The moisture sensor controlling apparatus 7 selects an operation state of the moisture sensor 1 on the basis of at least one of the position information regarding a position of the crop 8 or the water supply information regarding water supply to the soil 80, and on the basis of position information regarding a position of the moisture sensor 1.

The moisture sensor controlling apparatus 7 includes a communication section 71, an environment data processor 72, a sensor state selecting section 73, and the database 74. The moisture sensor controlling apparatus 7 includes, for example, a cloud server.

Each structural element is described in detail below.

### (Communication Section)

The communication section 71 is capable of communicating with an external apparatus including the control apparatus 6, and performs transmission and reception of various information. Specifically, the communication section 71 is capable of receiving respective pieces of environment data from the camera 41, the positioning section 43, the atmospheric-pressure sensor 44, the water spraying apparatus 42, the moisture amount measuring apparatus 10, and the weather server 9. The received environment data is output to the environment data processor 72.

The communication section 71 receives, from the camera 41, image data that is environment data.

The communication section 71 receives, from the positioning section 43 and the atmospheric-pressure sensor 44, pieces of position data of a position of each moisture sensor 1 that are pieces of environment data.

The communication section 71 receives, from the water spraying apparatus 42, water supply data that is environment data, the water supply data being data of water supply to the soil 80 that is performed by the water spraying apparatus 42.

The communication section 71 receives rainfall data that is provided by the weather server 9, the rainfall data being data of rainfall at a location at which soil is situated. Water supply data of water supply to soil due to rainfall can be acquired from the rainfall data. The rainfall data includes a time of starting rainfall, a time of stop of the rainfall, and an area, in soil, that has rainfall. Further, the rainfall data includes current-rainfall data and expected-rainfall data. For example, when moisture-sensor-installation-target soil has a large area, there is a possibility that it will rain locally without it raining in the entirety of the target soil for the same period of time. The rainfall data makes it possible to grasp in which area in target soil and for which period of time it is raining or it is expected to rain.

### (Environment Data Processor)

The environment data processor 72 calculates environment information regarding an environment of soil on the basis of environment data acquired through the communication section 71. The calculated environment information is notified (output) to the sensor state selecting section 73.

The environment information includes environment information regarding an environment of the soil 80 and change information regarding a change in the soil 80.

The environment information regarding an environment of the soil 80 includes position information regarding a position of the crop 8 and water supply information regarding water supply to the soil 80.

The environment data processor 72 acquires, through the communication section 71, image data acquired by the camera 41. The environment data processor 72 calculates position information regarding a position of the crop 8 from the image data.

As illustrated in Fig. 4, the position information regarding a position of the crop 8 includes position information regarding a position of a portion, such as a leaf 81 or a stem 83 of the crop 8, that is situated above the ground, and position information regarding a position of a portion, such as a root 82 of the crop 8, that is situated in the ground (in the soil 80) .

As position information regarding a portion of the crop 8 that is situated above the ground, the environment data processor 72 can calculate, from image data, position information regarding a position of a portion such as the leaf 81 or the stem 83.

Further, referring to growth information regarding growth of the crop 8 that is stored in advance in the database 74 described later, the environment data processor 72 can estimate a spreading range of the root 82 from an image of the stem 83 and the leaf 81 of the crop 8 that are situated above the ground, the spreading range of the root 82 being position information regarding a position of a portion of the crop 8 that is situated in the soil 80. From the spreading range of the root 82, a location of a tip of the root 82, that is, a location that is reached by the root 82 can be estimated.

The environment data processor 72 acquires, through the communication section 71, water supply data of water supply to the soil 80 that is performed by the water spraying apparatus 42, and water supply data of water supply to soil due to rainfall (rainfall data).

Using these pieces of data, the environment data processor 72 calculates water supply information regarding water supply to the soil 80. The water supply information regarding water supply to the soil 80 includes a moisture map that indicates a location of an area (a water supply area), in target soil, that is supplied with water, and water-supply-period-of-time information regarding a period of time for which water is supplied to the water supply area. The water-supply-period-of-time information includes, for example, a time of starting supplying water, a time of stopping supplying water, and a period of time for which water is supplied. It can also be said that the moisture map is water-supply-area information.

An example in which water supply information regarding water supply to the soil 80 is calculated using water supply data of water supply performed by the water spraying apparatus 42 and using the rainfall data, is described here, although the present technology is not limited thereto.

For example, the moisture map may be generated using detection data of detection performed by a plurality of moisture sensors 1 installed in the soil 80, the plurality of moisture sensors 1 being a portion of the moisture sensor controlling system 100. Further, a moisture sensor that is a portion of the moisture sensor controlling system 100, or a moisture sensor that is provided separately from the moisture sensor controlling system 100 may be used as the moisture sensor used to calculate water supply information. The case in which a moisture sensor is provided separately from the moisture sensor controlling system 100 is discussed. For example, a plurality of the moisture sensors is provided near the surface of the ground in the entirety of the target soil 80. The moisture map indicating a location of a water supply area, and the water-supply-period-of-time information regarding a period of time for which water is supplied to the water supply area can be obtained from installation position information regarding a position at which each moisture sensor is installed and from a temporal change in detection data of detection performed by the moisture sensor.

The environment data processor 72 acquires, through the communication section 71, water supply data of water supply to the soil 80 that is performed by the water spraying apparatus 42, and water supply data of water supply to soil due to rainfall (rainfall data), and acquires soil classification information and soil-water-moving-speed information from the database 74. Using these pieces of information, the environment data processor 72 estimates and calculates a soil water depth. Soil-water-depth information is included in the water supply information regarding water supply to the soil 80. It can also be said that the soil water depth is information regarding a location that soil water reaches.

An estimated soil water depth includes not only soil water spreading in the depth direction but also soil water spreading in the horizontal direction, and indicates a three-dimensional soil-water-permeation region in the soil 80.

Soil water after rainfall or spraying of water permeates into the soil at a different speed according to a soil environment indicated by, for example, soil classification. Further, soil water does not necessarily permeate in one direction, and the environment data processor 72 estimates a current soil water depth using the soil classification information and the soil-water-moving-speed information.

Further, the soil-water-depth information may include an estimated temporal change in soil-water-permeation region. When, for example, water is supplied to the soil 80 from the soil surface due to, for example, spraying of water or rainfall, the soil-water-permeation region is changed as time goes on, such as the case in which soil water gradually permeates broadly into the soil as time goes on. The environment data processor 72 may calculate an estimated temporal change in soil-water-permeation region on the basis of the soil classification and the soil-water-moving-speed information.

The environment data processor 72 may receive, through the communication section 71, pieces of position data of positions of each moisture sensor 1 that are respectively detected by the positioning section 43 and the atmospheric-pressure sensor 44, and may calculate position information regarding a position of the moisture sensor 1 in the soil 80, using the pieces of received position data.

### (Sensor State Selecting Section)

The sensor state selecting section 73 refers to environment information notified by the environment data processor 72, and controls an operation state of each moisture sensor 1. More specifically, the sensor state selecting section 73 determines an operation state of each moisture sensor 1 on the basis of at least one of position information regarding a position of the crop 8 or water supply information regarding water supply to the soil 80, and on the basis of position information regarding a position of the moisture sensor 1, and controls the moisture sensor 1 such that the moisture sensor 1 is in the determined operation state.

Position information that is stored in the database 74 in advance may be used as the position information regarding a position of the moisture sensor 1. Alternatively, the position information regarding a position of each moisture sensor 1 may be calculated using pieces of position data that are respectively generated by the positioning section 43 and the atmospheric-pressure sensor 44.

A specific method for controlling a moisture sensor by selecting an operation state of the moisture sensor will be described later.

### (Database)

The database 74 stores therein position information regarding a position of each moisture sensor 1, soil classification information, soil-water-moving-speed information, and growth information regarding growth of a crop. These pieces of information may be acquired and stored in advance.

The position information regarding a position of the moisture sensor 1 is information that uniquely indicates a position of the moisture sensor 1 in soil.

The soil classification information indicates soil classification for soil in which a control-target moisture sensor is installed.

The soil water moving speed refers to a speed at which water in soil moves. The soil water moving speed differs depending on the soil classification, and is information that can be acquired in advance according to the soil classification. When information regarding the soil water moving speed is not allowed to be acquired in advance, the soil water moving speed can be estimated from the soil classification and a moisture amount in soil that is calculated by the moisture amount measuring apparatus 10.

The growth information regarding growth of the crop 8 can be acquired in advance using, for example, machine learning.

The growth information regarding growth of the crop 8 includes, for example, image data and spreading-range information that are associated with each other for each stage of growth of the crop 8, the image data being data of an image that includes portions, such as the stem 83 and the leaf 81, that are situated above the ground, the spreading-range information being information regarding a spreading range of the root 82 situated in soil. Referring to the growth information regarding growth of the crop 8 being stored in the database, position information regarding a position of a root, such as a spreading range of a root of the crop that is situated in soil, can be estimated from image data of a portion of the crop that is situated above the ground.

Further, a height of a portion, of the crop 8, that is situated above the ground; information regarding a leaf such as the number of leaves 81, and a size, a shape, and a color of the leaf 81; and a spreading range of the root 82 situated in soil may be associated with each other, and may be stored in the database as the growth information regarding growth of the crop 8. The height of the crop 8, and the information regarding a leaf such as the number of leaves 81, and a size, a shape, and a color of the leaf 81 may be estimated from image data of the crop 8, where the information regarding a leaf is estimated by a region of leaves being extracted. The position information regarding a position of a portion of the crop that is situated in the soil can be estimated from a result of the estimation described above and the growth information stored in the database.

### <Moisture Sensor Controlling Method>

Examples of a method for controlling a moisture sensor by processing of selecting an operation state of the moisture sensor being performed are described in embodiments below, the method for controlling a moisture sensor being performed by the moisture sensor controlling system 100 described above. In the following embodiments, an example of using position information regarding a position of a moisture sensor that is stored in the database 74 in advance is described. Processing of selecting an operation state of a moisture sensor that is described in the embodiments is performed for, for example, each specified period of time. Note that the frequency in processing can be set as appropriate.

### [First Embodiment]

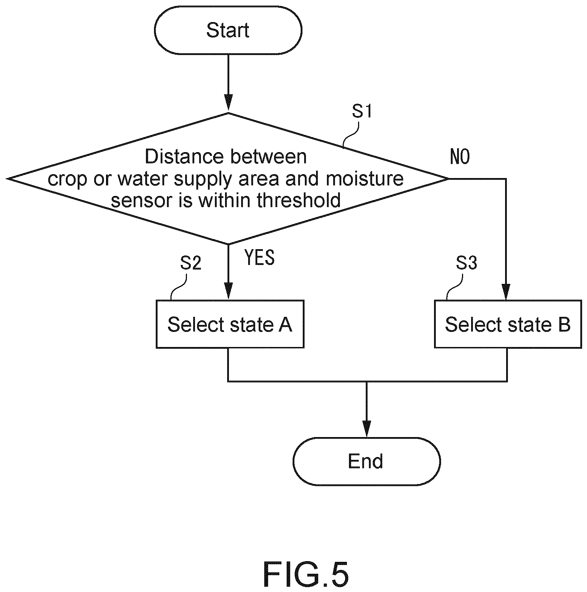

Fig. 5 is a flowchart of a moisture sensor controlling method according to a first embodiment. An example illustrated in Fig. 5 is an example of controlling an operation state of the moisture sensor 1 by selecting the state A or the state B as the operation state of the moisture sensor 1, on the basis of position information regarding a position of the crop 8 or water supply information regarding water supply to the soil 80, and on the basis of position information regarding a position of the moisture sensor 1.

As described above, the state A is a detection state in which power is supplied, and the state B is a detection stop state in which no power is supplied.

### (Example of Controlling Operation State of Moisture Sensor on Basis of Position Information Regarding Position of Crop and Position Information Regarding Position of Moisture Sensor)

First, an example of controlling an operation state of each moisture sensor 1 on the basis of position information regarding a position of the crop 8 that is environment information regarding an environment of the soil 80, and on the basis of position information regarding a position of the moisture sensor 1, is described.

When processing of controlling a moisture sensor is started, the environment data processor 72 of the moisture sensor controlling apparatus 7 acquires, from the control apparatus 6, image data that is acquired by the camera 41, and calculates position information regarding a position of the crop 8 using the image data. The position information regarding a position of the crop 8 is output to the sensor state selecting section 73.

In the present embodiment, an example in which position information regarding a position of the stem 83 situated above the ground in a horizontal plane (the XY plane) obtained when soil is viewed planarly in the depth direction is used as the position information regarding a position of a portion of the crop 8, is described. Note that not the position information regarding a position of the stem 83 but position information regarding a position of the leaf 81 may be used as the position information regarding a position of a portion of the crop 8. Further, position information regarding a position of the root 82 situated in the soil 80 in the depth direction may be used as the position information regarding a position of a portion of the crop 8. This will be described in another embodiment.

As illustrated in Fig. 5, the sensor state selecting section 73 of the moisture sensor controlling apparatus 7 determines whether a distance between the moisture sensor 1 and the stem 83 of the crop 8 in the horizontal plane obtained when soil is viewed planarly in the depth direction, is within a threshold, using the position information regarding a position of the stem 83 of the crop 8, and using the position information regarding a position of the moisture sensor 1 being stored in the database 74 (S1).

When the sensor state selecting section 73 determines that the distance is within the threshold (YES), the sensor state selecting section 73 selects the state A (S2). When the sensor state selecting section 73 determines that the distance is outside of the threshold (NO), the sensor state selecting section 73 selects the state B (S3).

Information regarding the selected sensor state is transmitted to the control apparatus 6 through the communication section 71. The sensor controller 61 of the control apparatus 6 controls the moisture sensor 1 on the basis of the received sensor state information. The processing illustrated in Fig. 4 is performed for each moisture sensor 1.

In the present embodiment, the moisture sensor 1 of which a distance to the crop 8 is outside of the threshold can be set to be in a detection stop state, as described above. In other words, there is no need to monitor a moisture amount in soil in an area in which the moisture sensor 1 situated away from the crop 8 is installed. Thus, control is performed to stop sensing performed by a moisture sensor in such an area.

On the other hand, only the moisture sensor 1 of which a distance to the crop 8 is within the threshold, that is, only the moisture sensor 1 situated in an area for which there is a need to monitor a moisture amount in soil is operated and set to be in a detection state.

This makes it possible to selectively operate a moisture sensor, from among a plurality of moisture sensors, that is situated in an area for which there is estimated to be a need to monitor a moisture amount in soil. This results in being able to reduce power consumption of each moisture sensor 1, and thus to reduce power consumption of the entirety of the moisture sensor controlling system 100.

### (Example of Controlling Operation State of Moisture Sensor on Basis of Water Supply Information Regarding Water Supply to Soil and Position Information Regarding Position of Moisture Sensor)

Next, an example of controlling the operation state of each moisture sensor 1 on the basis of water supply information regarding water supply to the soil 80 that is the environment information, and on the basis of the position information regarding a position of the moisture sensor 1, is described.

When processing of controlling a moisture sensor is started, the environment data processor 72 of the moisture sensor controlling apparatus 7 acquires water supply data of water supply performed by the water spraying apparatus 42 from the control apparatus 6, and acquires rainfall data from the weather server 9. The environment data processor 72 calculates water supply information regarding water supply to the soil 80 using these pieces of data. The calculated water supply information is output to the sensor state selecting section 73.

The water supply information includes water-supply-area information and water-supply-period-of-time information regarding a period of time for which water is supplied to a water supply area.

Next, as illustrated in Fig. 5, the sensor state selecting section 73 of the moisture sensor controlling apparatus 7 determines whether a distance between the moisture sensor 1 and the water supply area in the horizontal plane obtained when soil is viewed planarly in the depth direction, is within a threshold, using the water supply information regarding water supply to the soil 80, and using the position information regarding a position of the moisture sensor 1 being stored in the database 74 (S1).

When the sensor state selecting section 73 determines that the distance is within the threshold (YES), the sensor state selecting section 73 selects the state A (S2). The state in which "the distance between the moisture sensor 1 and the water supply area is within the threshold" includes a state in which the moisture sensor 1 is situated in the water supply area.

When the sensor state selecting section 73 determines that the distance is outside of the threshold (NO), the sensor state selecting section 73 selects the state B (S3).

Here, there is expected to be a rapid change in a moisture amount in soil when water is supplied to the soil 80. Thus, there is a need for detection performed by the moisture sensor 1 in order to track the change. On the other hand, the moisture amount in soil is hardly changed when water is not supplied to the soil 80. Thus, there is not necessarily a need for detection performed by the moisture sensor 1.

In the present embodiment, control is performed to stop sensing performed by the moisture sensor 1 situated in an area in which a moisture amount in soil is estimated to be hardly changed.

The water supply information includes water-supply-area information and water-supply-period-of-time information regarding a period of time for which water is supplied to a water supply area. In other words, the water supply information regarding water supply to the soil 80 may include an estimated temporal change in whether water is supplied to an area in which each moisture sensor is situated in the soil 80 for a specified period of time.

Using the information described above, the sensor state selecting section 73 can control the moisture sensor 1 such that an operation state of the moisture sensor 1 is changed to an appropriate state at an appropriate timing.

When a plurality of moisture sensors is installed in soil having a large area, whether water is supplied may depend on a period of time, and may differ depending on the location at which a moisture sensor is installed. For example, an example in which water is sprayed for thirty minutes over soil that includes a first area and a second area is described. When water is sprayed for the first fifteen minutes only on the first area and when water is sprayed for the last fifteen minutes only on the second area, the change in whether water is supplied to an area for the period of time of 30 minutes, differs depending on the area.

In such a case, control can be performed on each moisture sensor in the present embodiment, such that only a moisture sensor situated in the first area is set to be in a detection state for the first fifteen minutes and such that only a moisture sensor situated in the second area is set to be in the detection state for the last fifteen minutes. In other words, control can be performed on the basis of water supply information regarding water supply to an area in which a moisture sensor is situated, such that the operation state of the moisture sensor is set to be an appropriate state at an appropriate timing.

Information regarding the selected sensor state is transmitted to the control apparatus 6 through the communication section 71. The sensor controller 61 of the control apparatus 6 controls the moisture sensor 1 on the basis of the received sensor state information. The processing illustrated in Fig. 5 is performed for each moisture sensor 1.

In the present embodiment, the moisture sensor 1 of which a distance to a water supply area is outside of the threshold is set to be in a detection stop state, as described above. Then, only the moisture sensor 1 situated in an area in which there is, or there is expected to be a rapid change in a moisture amount in soil that is caused due to water being supplied to the soil 80, that is, in an area for which there is a need to monitor a moisture amount in soil, is operated and set to be in a detection state.

This makes it possible to selectively operate a moisture sensor, from among a plurality of moisture sensors, that is situated in an area for which there is estimated to be a need to monitor a moisture amount in soil, and to set the selectively operated moisture sensor to be in a detection state. This results in being able to reduce power consumption of each moisture sensor 1, and thus to reduce power consumption of the entirety of the moisture sensor controlling system 100.

The example in which an operation state of the moisture sensor is controlled using, as environment information, one of position information regarding a position of a crop and water supply information regarding water supply to soil, has been described in the first embodiment. An example in which the operation state of the moisture sensor is controlled using both position information regarding a position of a crop and water supply information regarding water supply to soil, is described in second to fourth embodiments below.

### [Second Embodiment]

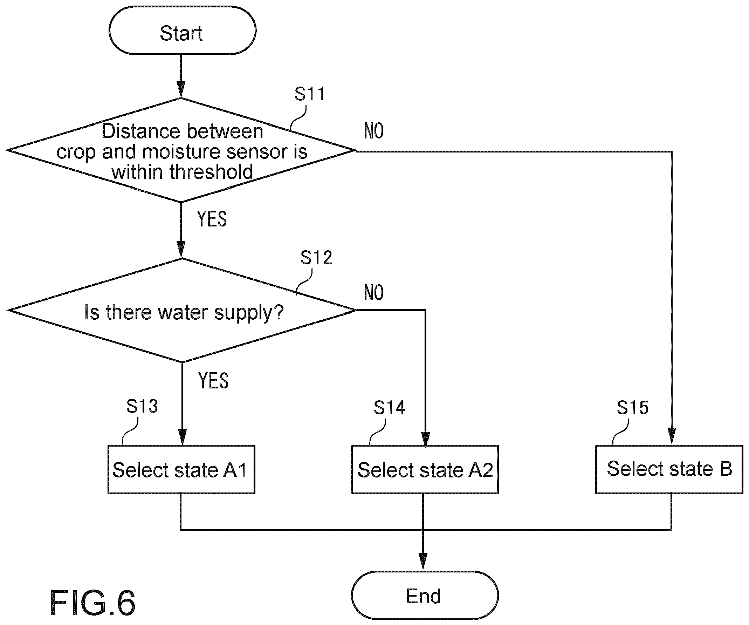

Fig. 6 is a flowchart of the moisture sensor controlling method according to a second embodiment. An example illustrated in Fig. 6 is an example of controlling an operation state of the moisture sensor 1 by selecting the operation state of the moisture sensor 1 from the state Al, the state A2, and the state B, on the basis of position information regarding a position of the crop 8, water supply information regarding water supply to the soil 80, and position information regarding a position of the moisture sensor 1.

As described above, the state A1 is a frequent-detection state. The state A2 is a less-frequent-detection state. The state B is a detection stop state.

When processing of controlling a moisture sensor is started, the environment data processor 72 of the moisture sensor controlling apparatus 7 acquires, from the control apparatus 6, image data that is acquired by the camera 41, and calculates position information regarding a position of the crop 8 using the image data. The position information regarding a position of the crop 8 is output to the sensor state selecting section 73. In the present embodiment, an example in which position information regarding a position of the stem 83 in the horizontal plane obtained when soil is viewed planarly in the depth direction is used, is described.

Further, the environment data processor 72 acquires water supply data of water supply performed by the water spraying apparatus 42 from the control apparatus 6, and acquires rainfall data from the weather server 9. The environment data processor 72 calculates water supply information regarding water supply to the soil 80 using these pieces of information. The calculated water supply information is output to the sensor state selecting section 73.

Next, as illustrated in Fig. 6, the sensor state selecting section 73 of the moisture sensor controlling apparatus 7 determines whether a distance between the moisture sensor 1 and the stem 83 of the crop 8 in the horizontal plane obtained when soil is viewed planarly in the depth direction, is within a threshold, using the position information regarding a position of the stem 83 of the crop 8, and using the position information regarding a position of the moisture sensor 1 being stored in the database 74 (S11).

When the sensor state selecting section 73 determines that the distance is within the threshold (YES), the process moves on to S12. When the sensor state selecting section 73 determines that the distance is outside of the threshold (NO), the sensor state selecting section 73 selects the state B (S15).

In S12, on the basis of the water supply information, the sensor state selecting section 73 determines whether there is water supply at a location at which the moisture sensor 1 is situated, that is, whether the moisture sensor 1 is situated in the water supply area.

When the sensor state selecting section 73 determines that there is water supply (YES), the sensor state selecting section 73 selects the state A1 (S13). The state in which there is water supply is a state in which there is expected to be a rapid change in a moisture amount in soil. Thus, the state A1 corresponding to a frequent-detection state is selected in order to track the change.

When the sensor state selecting section 73 determines that there is no water supply (NO), the sensor state selecting section 73 selects the state A2 (S14). The state in which there is no water supply is a state in which there is not expected to be a rapid change in a moisture amount in soil. Thus, the state A2 corresponding to a less-frequent-detection state is selected.

This makes it possible to improve the detection accuracy and to reduce power consumption.

Further, the sensor state selecting section 73 controls an operation state of each moisture sensor on the basis of an estimated temporal change in whether water is supplied at a location at which the moisture sensor is situated in the soil 80 for a specified period of time (the water supply information). This makes it possible to control each moisture sensor 1 such as changing an operation state of the moisture sensor 1 to an appropriate state at an appropriate timing.

Information regarding the selected sensor state is transmitted to the control apparatus 6 through the communication section 71. The sensor controller 61 of the control apparatus 6 controls the moisture sensor 1 on the basis of the received sensor state information. The processing illustrated in Fig. 6 is performed for each moisture sensor 1.

In the present embodiment, the moisture sensor 1 of which a distance to a portion of the crop 8 that is situated above the ground in the horizontal plane is outside of the threshold is set to be in a detection stop state in which no detection is performed, as described above. Then, from among the moisture sensors 1 of which the distance to a portion of the crop 8 that is situated above the ground is within the threshold, the moisture sensor 1 situated in an area in which there is water supply is set to be in a frequent-detection state, and the moisture sensor 1 situated in an area in which there is no water supply is set to be in a less-frequent-detection state.

This makes it possible to selectively operate a moisture sensor, from among a plurality of moisture sensors, that is situated in an area for which there is estimated to be a need to monitor a moisture amount in soil, and to set the selectively operated moisture sensor to be in a detection state. Further, control performed on the frequency in detection performed by the operated moisture sensor results in being able to reduce power consumption of each moisture sensor 1, and thus to reduce power consumption of the entirety of the moisture sensor controlling system 100.

### [Third Embodiment]

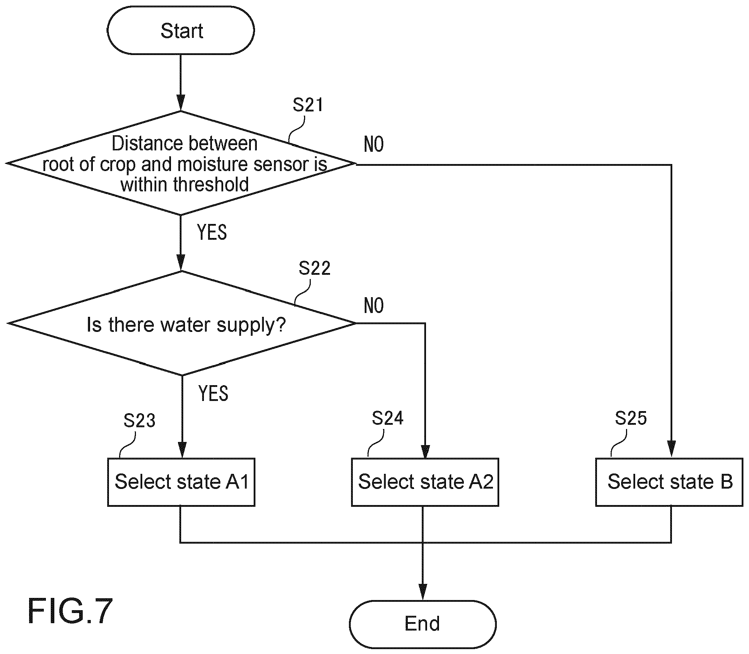

Fig. 7 is a flowchart of the moisture sensor controlling method according to a third embodiment. As in the second embodiment, an example illustrated in Fig. 7 is an example of controlling an operation state of the moisture sensor 1 by selecting the operation state of the moisture sensor 1 from the state A1, the state A2, and the state B, on the basis of position information regarding a position of the crop 8, water supply information regarding water supply to the soil 80, and position information regarding a position of the moisture sensor 1.

The third embodiment is different from the second embodiment only in that position information regarding a position of the root 82, of the crop 8, that is situated in the soil 80 is used as the position information regarding a position of the crop 8.

When processing of controlling a moisture sensor is started, the environment data processor 72 of the moisture sensor controlling apparatus 7 acquires, from the control apparatus 6, image data that is acquired by the camera 41. The environment data processor 72 refers to growth information regarding growth of the crop that is stored in the database 74 to estimate a spreading range of the root situated in the soil 80 using the image data, and calculates the position information regarding a position of the root 82. The position information regarding a position of the root 82 of the crop 8 is output to the sensor state selecting section 73.

Further, the environment data processor 72 acquires water supply data of water supply performed by the water spraying apparatus 42 from the control apparatus 6, and acquires rainfall data from the weather server 9. The environment data processor 72 calculates water supply information regarding water supply to the soil 80 using these pieces of information. The calculated water supply information is output to the sensor state selecting section 73.

In the present embodiment, position information regarding a position of the root 82 corresponding to a portion of the crop 8 that is situated in the soil 80 is used as the position information regarding a position of the crop 8. For example, the position information regarding a position of the root 82 is calculated as a spreading range of the root 82 in a three-dimensional space in soil.

In the present embodiment, not only position information in the horizontal plane but also position information in the depth direction (the vertical direction) is considered the position information regarding a position of the crop 8 to control each moisture sensor, as described above. This makes it possible to more accurately control a sensor state of each moisture sensor, and thus to reduce power consumption of the entirety of the moisture sensor controlling system 100.

Next, as illustrated in Fig. 7, the sensor state selecting section 73 of the moisture sensor controlling apparatus 7 determines whether a distance between the moisture sensor 1 and the root 82 of the crop 8 is within a threshold, using the position information regarding a position of the root 82 of the crop 8, and using the position information regarding a position of the moisture sensor 1 being stored in the database 74 (S21). Specifically, the determination can be performed using a distance between a tip of the root 82 and the moisture sensor 1, the tip of the root 82 indicating a location that is reached by the root 82 in the soil 80.

When the sensor state selecting section 73 determines that the distance is within the threshold (YES), the process moves on to S22. When the sensor state selecting section 73 determines that the distance is outside of the threshold (NO), the sensor state selecting section 73 selects the state B (S25).

In S22, on the basis of the water supply information, the sensor state selecting section 73 determines whether there is water supply at a location at which the moisture sensor 1 is situated, that is, whether the moisture sensor 1 is situated in the water supply area.

When the sensor state selecting section 73 determines that there is water supply (YES), the sensor state selecting section 73 selects the state A1 (S23). The state in which there is water supply is a state in which there is expected to be a rapid change in a moisture amount in soil. Thus, the state A1 corresponding to a frequent-detection state is selected in order to track the change.

When the sensor state selecting section 73 determines that there is no water supply (NO), the sensor state selecting section 73 selects the state A2 (S24). The state in which there is no water supply is a state in in which there is not expected to be a rapid change in a moisture amount in soil. Thus, the state A2 corresponding to a less-frequent-detection state is selected.

This makes it possible to improve the detection accuracy and to reduce power consumption.

Information regarding the selected sensor state is transmitted to the control apparatus 6 through the communication section 71. The sensor controller 61 of the control apparatus 6 controls the moisture sensor 1 on the basis of the received sensor state information. The processing illustrated in Fig. 7 is performed for each moisture sensor 1.

In the present embodiment, the moisture sensor 1 of which a distance to a portion of the crop 8 that is situated in the soil 80 is outside of the threshold is set to be in a detection stop state, as described above. From among the moisture sensors 1 of which the distance is within the threshold, the moisture sensor 1 situated in an area in which there is water supply is set to be in a frequent-detection state, and the moisture sensor 1 situated in an area in which there is no water supply is set to be in a less-frequent-detection state.

This makes it possible to only operate a moisture sensor, from among a plurality of moisture sensors, that is situated in an area for which there is estimated to be a need to monitor a moisture amount in soil, and to set the operated moisture sensor to be in a detection state. Further, control performed on the frequency in detection performed by the operated moisture sensor results in being able to reduce power consumption of each moisture sensor 1, and thus to reduce power consumption of the entirety of the moisture sensor controlling system 100.

In the present embodiment, the use of a positional relationship between a moisture sensor and a root of a crop makes it possible to control an operation state of the moisture sensor flexibly and optimally according to a degree of growth of the crop.

### [Fourth Embodiment]

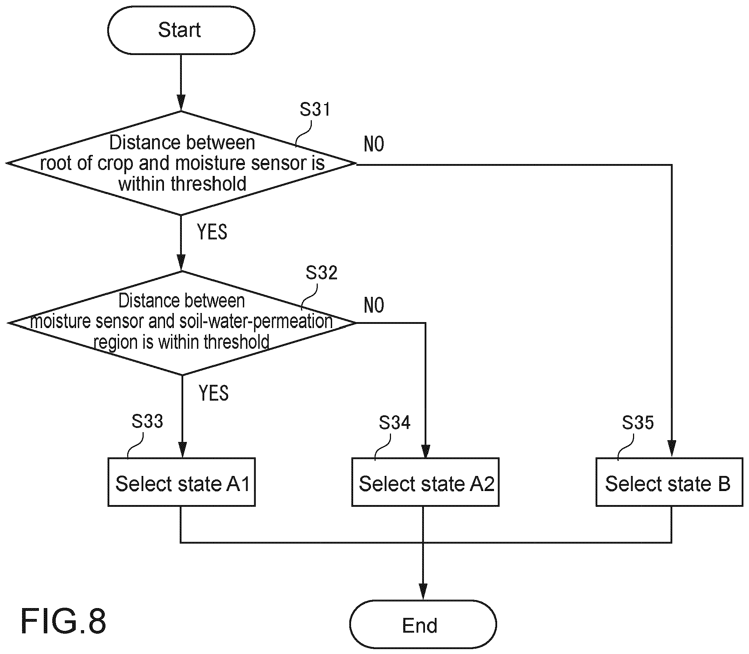

Fig. 8 is a flowchart of the moisture sensor controlling method according to a fourth embodiment. An example illustrated in Fig. 8 is an example of controlling an operation state of the moisture sensor 1 by selecting the operation state of the moisture sensor 1 from the state A1, the state A2, and the state B, on the basis of position information regarding a position of the root 82 of the crop 8, water supply information regarding water supply to the soil 80, and position information regarding a position of the moisture sensor 1.

In the present embodiment, a soil-water-permeation region (soil-water-depth information) is used as the water supply information regarding water supply to the soil 80. Instead of determining whether there is water supply (S22) in the third embodiment, the present embodiment includes determining whether a distance between the soil-water-permeation region and the moisture sensor is within a threshold.

This is described below.

When processing of controlling a moisture sensor is started, the environment data processor 72 of the moisture sensor controlling apparatus 7 acquires, from the control apparatus 6, image data that is acquired by the camera 41, and calculates position information regarding a position of the root 82 of the crop 8 using the image data. The position information regarding a position of the root 82 of the crop 8 is output to the sensor state selecting section 73.

Further, the environment data processor 72 acquires water supply data of water supply performed by the water spraying apparatus 42 from the control apparatus 6, acquires rainfall data from the weather server 9, and acquires soil classification information and soil-water-moving-speed information from the database 74. The environment data processor 72 estimates and calculates a soil-water-permeation region using these pieces of information.

Information regarding the calculated soil-water-permeation region is output to the sensor state selecting section 73.

Next, as illustrated in Fig. 8, the sensor state selecting section 73 of the moisture sensor controlling apparatus 7 determines whether a distance between the moisture sensor 1 and the root 82 of the crop 8 is within a threshold, using the position information regarding a position of the root 82 of the crop 8, and using the position information regarding a position of the moisture sensor 1 being stored in the database 74 (S31).

When the sensor state selecting section 73 determines that the distance is within the threshold (YES), the process moves on to S32.

When the sensor state selecting section 73 determines that the distance is outside of the threshold (NO), the sensor state selecting section 73 selects the state B (S35).

In S32, the sensor state selecting section 73 determines whether a distance between the soil-water-permeation region and the moisture sensor 1 is within a threshold.

When the sensor state selecting section 73 determines that the distance is within the threshold (YES), the sensor state selecting section 73 selects the state A1 (S33). The state in which the distance between the soil-water-permeation region and the moisture sensor 1 is within the threshold refers to a state in which an area for which there is expected to be a rapid change in a moisture amount in soil is situated near the moisture sensor. Thus, the state A1 corresponding to a frequent-detection state is selected in order to track the change in a moisture amount in soil.

When the sensor state selecting section 73 determines that the distance is outside of the threshold (NO), the sensor state selecting section 73 selects the state A2 (S34). The state in which the distance between the soil-water-permeation region and the moisture sensor 1 is outside of the threshold refers to a state in which an area for which there is expected to be a rapid change in a moisture amount in soil is situated away from the moisture sensor. Thus, the state A2 corresponding to a less-frequent-detection state is selected.

Further, on the basis of information regarding an estimated temporal change in the soil-water-permeation region calculated by the environment data processor 72, the sensor state selecting section 73 can control each moisture sensor 1 such as changing an operation state of the moisture sensor 1 to an appropriate state at an appropriate timing.

As described above, control performed on a timing of operating the moisture sensor 1 on the basis of a soil water permeating speed makes it possible to control operation information regarding an operation of a moisture sensor in more detail, and to reduce power consumption efficiently.

Information regarding the selected sensor state is transmitted to the control apparatus 6 through the communication section 71. The sensor controller 61 of the control apparatus 6 controls the moisture sensor 1 on the basis of the received sensor state information. The processing illustrated in Fig. 7 is performed for each moisture sensor 1.

In the present embodiment, the moisture sensor 1 of which a distance to a portion of the crop 8 that is situated in the soil 80 is outside of the threshold is set to be in a detection stop state, as described above. Further, from among the moisture sensors 1 of which the distance to the portion, of the crop 8, that is situated in the soil 80 is within the threshold, the moisture sensor 1 of which a distance to a soil-water-permeation region is within a threshold is set to be in a frequent-detection state, and the moisture sensor 1 of which the distance to the soil-water-permeation region is outside of the threshold is set to be in a less-frequent-detection state.

This makes it possible to selectively operate a moisture sensor, from among a plurality of moisture sensors, that is situated in an area for which there is estimated to be a need to monitor a moisture amount in soil. Further, control performed in detail on a sensor state of the operated moisture sensor results in being able to reduce power consumption of each moisture sensor 1, and thus to reduce power consumption of the entirety of the moisture sensor controlling system 100.

The present embodiment makes it possible to optimally control an operation state of the moisture sensor according to the movement of soil water.

The example in which whether detection is to be performed by the sensor section of the moisture sensor and how frequent detection is to be performed are controlled by a state of power supply to the signal generator 31 being controlled, and this results in reducing power consumption, has been described in the embodiments above. An example in which a state of supplying the communication section 32 with transmission power used to transmit detection data detected by the moisture sensor is controlled to reduce power consumption, is described in fifth to eighth embodiments below. The respective embodiments are described below.

### [Fifth Embodiment]

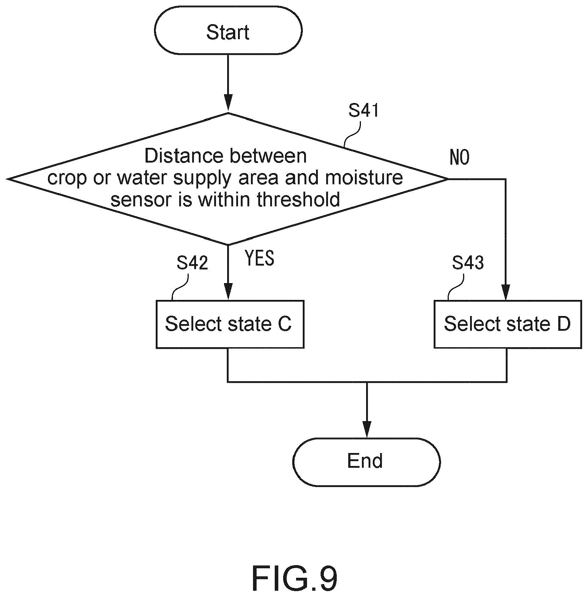

Fig. 9 is a flowchart of a moisture sensor controlling method according to a fifth embodiment. The fifth embodiment is different from the first embodiment only in that options of an operation state of the moisture sensor are different, and a basic flow of the fifth embodiment is similar to that of the first embodiment.

An example illustrated in Fig. 9 is an example of controlling an operation state of the moisture sensor 1 by selecting the state C or the state D as the operation state of the moisture sensor 1, on the basis of position information regarding a position of the crop 8 or water supply information regarding water supply to the soil 80, and on the basis of position information regarding a position of the moisture sensor 1.

As described above, the state C is a communication state in which transmission power is supplied, and the state D is a communication stop state in which no transmission power is supplied.

### (Example of Controlling Operation State of Moisture Sensor on Basis of Position Information Regarding Position of Crop and Position Information Regarding Position of Moisture Sensor)

First, an example of controlling an operation state of each moisture sensor 1 on the basis of position information regarding a position of the crop 8 that is the environment information, and on the basis of position information regarding a position of the moisture sensor 1, is described.

When processing of controlling a moisture sensor is started, the environment data processor 72 of the moisture sensor controlling apparatus 7 acquires, from the control apparatus 6, image data that is acquired by the camera 41, and calculates position information regarding a position of the crop 8 using the image data. The position information regarding a position of the crop 8 is output to the sensor state selecting section 73.

In the present embodiment, an example in which position information regarding a position of the stem 83 in the horizontal plane obtained when soil is viewed planarly in the depth direction is used as the position information regarding a position of the crop 8, is described.

As illustrated in Fig. 9, the sensor state selecting section 73 of the moisture sensor controlling apparatus 7 determines whether a distance between the moisture sensor 1 and the stem 83 of the crop 8 in the horizontal plane obtained when soil is viewed planarly in the depth direction, is within a threshold, using the position information regarding a position of the stem 83 of the crop 8, and using the position information regarding a position of the moisture sensor 1 being stored in the database 74 (S41).

When the sensor state selecting section 73 determines that the distance is within the threshold (YES), the sensor state selecting section 73 selects the state C (S42).

When the sensor state selecting section 73 determines that the distance is outside of the threshold (NO), the sensor state selecting section 73 selects the state D (S43).

Information regarding the selected sensor state is transmitted to the control apparatus 6 through the communication section 71. The sensor controller 61 of the control apparatus 6 controls the moisture sensor 1 on the basis of the received sensor state information. The processing illustrated in Fig. 9 is performed for each moisture sensor 1.

In the present embodiment, the moisture sensor 1 of which a distance to the crop 8 is outside of the threshold is set to be in a communication stop state, as described above. Further, the moisture sensor 1 of which a distance to the crop 8 is within the threshold, that is, the moisture sensor 1 situated in an area for which there is a need to monitor a moisture amount in soil is set to be in a communication state in which detection data of detection performed by the moisture sensor 1 can be transmitted.

Consequently, a moisture sensor, from among a plurality of moisture sensors, that is situated in an area for which there is estimated to be a need to monitor a moisture amount in soil can be selectively set to be in a communication state. This results in being able to reduce consumption of power used for communication performed by each moisture sensor 1, and thus to reduce power consumption of the entirety of the moisture sensor controlling system 100.

### (Example of Controlling Operation State of Moisture Sensor on Basis of Water Supply Information Regarding Water Supply to Soil and Position Information Regarding Position of Moisture Sensor)

Next, an example of controlling the operation state of each moisture sensor 1 on the basis of water supply information regarding water supply to the soil 80 that is the environment information, and on the basis of the position information regarding a position of the moisture sensor 1, is described.

When processing of controlling a moisture sensor is started, the environment data processor 72 of the moisture sensor controlling apparatus 7 acquires water supply data of water supply performed by the water spraying apparatus 42 from the control apparatus 6, and acquires rainfall data from the weather server 9. The environment data processor 72 calculates water supply information regarding water supply to the soil 80 using these pieces of data. The calculated water supply information is output to the sensor state selecting section 73.

The water supply information includes water-supply-area information and water-supply-period-of-time information regarding a period of time for which water is supplied to a water supply area.

Next, as illustrated in Fig. 9, the sensor state selecting section 73 of the moisture sensor controlling apparatus 7 determines whether a distance between the moisture sensor 1 and the water supply area in the horizontal plane obtained when soil is viewed planarly in the depth direction, is within a threshold, using the water supply information regarding water supply to the soil 80, and using the position information regarding a position of the moisture sensor 1 being stored in the database 74 (S41).

When the sensor state selecting section 73 determines that the distance is within the threshold (YES), the sensor state selecting section 73 selects the state C (S42). When the sensor state selecting section 73 determines that the distance is outside of the threshold (NO), the sensor state selecting section 73 selects the state D (S43).

Information regarding the selected sensor state is transmitted to the control apparatus 6 through the communication section 71. The sensor controller 61 of the control apparatus 6 controls the moisture sensor 1 on the basis of the received sensor state information. The processing illustrated in Fig. 9 is performed for each moisture sensor 1.

In the present embodiment, the moisture sensor 1 of which a distance to a water supply area is outside of the threshold is set to be in a communication stop state, as described above. Then, the moisture sensor 1 situated in an area in which there is, or there is expected to be a rapid change in a moisture amount in soil that is caused due to water being supplied to the soil 80, that is, in an area for which there is a need to monitor a moisture amount in soil, is set to be in a communication state in which detection data of detection performed by the moisture sensor 1 can be transmitted.

Consequently, a moisture sensor, from among a plurality of moisture sensors, that is situated in an area for which there is estimated to be a need to monitor a moisture amount in soil can be selectively set to be in a communication state. This results in being able to reduce consumption of power used for communication performed by each moisture sensor 1, and thus to reduce power consumption of the entirety of the moisture sensor controlling system 100.

The example in which an operation state of the moisture sensor is controlled using, as environment information, one of position information regarding a position of a crop and water supply information regarding water supply to soil, has been described in the fifth embodiment. An example in which the operation state of the moisture sensor is controlled using both position information regarding a position of a crop and water supply information regarding water supply to soil, is described in the sixth to eighth embodiments below.

### [Sixth Embodiment]

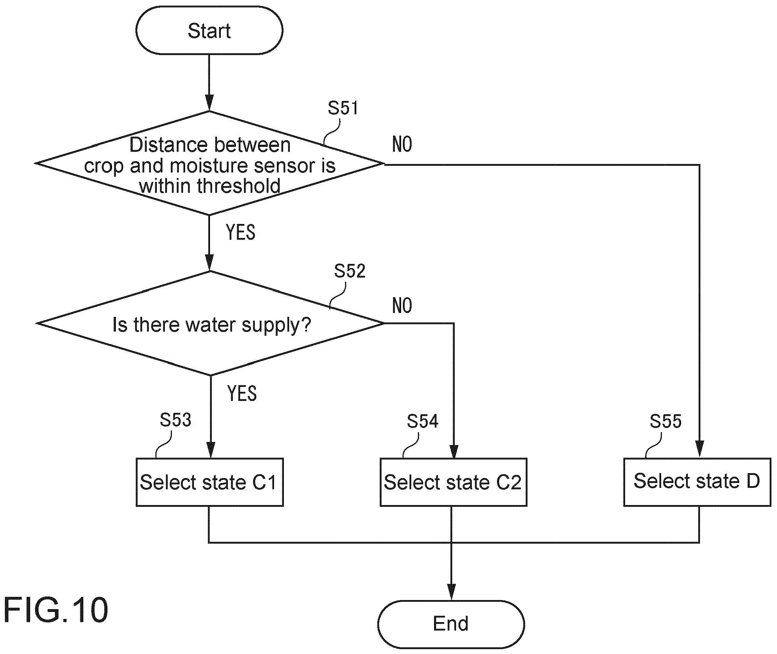

Fig. 10 is a flowchart of the moisture sensor controlling method according to a sixth embodiment. The sixth embodiment is different from the second embodiment only in that options of an operation state of the moisture sensor are different, and a basic flow of the sixth embodiment is similar to that of the second embodiment.

An example illustrated in Fig. 10 is an example of controlling an operation state of the moisture sensor 1 by selecting the operation state of the moisture sensor 1 from the state C1, the state C2, and the state D, on the basis of position information regarding a position of the crop 8, water supply information regarding water supply to the soil 80, and position information regarding a position of the moisture sensor 1.

As described above, the state C1 is a frequent-communication state. The state C2 is a less-frequent-communication state. The state D is a communication stop state.

When processing of controlling a moisture sensor is started, the environment data processor 72 of the moisture sensor controlling apparatus 7 acquires, from the control apparatus 6, image data that is acquired by the camera 41, and calculates position information regarding a position of the crop 8 using the image data. The position information regarding a position of the crop 8 is output to the sensor state selecting section 73. In the present embodiment, an example in which position information regarding a position of the stem 83 in the horizontal plane obtained when soil is viewed planarly in the depth direction is used, is described.

Further, the environment data processor 72 acquires water-spraying data of water spraying performed by the water spraying apparatus 42 from the control apparatus 6, and acquires rainfall data from the weather server 9. The environment data processor 72 calculates water supply information regarding water supply to the soil 80 using these pieces of information. The calculated water supply information is output to the sensor state selecting section 73.

Next, as illustrated in Fig. 10, the sensor state selecting section 73 of the moisture sensor controlling apparatus 7 determines whether a distance between the moisture sensor 1 and the stem 83 of the crop 8 in the horizontal plane obtained when soil is viewed planarly in the depth direction, is within a threshold, using the position information regarding a position of the stem 83 of the crop 8, and using the position information regarding a position of the moisture sensor 1 being stored in the database 74 (S51).

When the sensor state selecting section 73 determines that the distance is within the threshold (YES), the process moves on to S52. When the sensor state selecting section 73 determines that the distance is outside of the threshold (NO), the sensor state selecting section 73 selects the state D (S55).

In S52, on the basis of the water supply information, the sensor state selecting section 73 determines whether there is water supply at a location at which the moisture sensor 1 is situated, that is, whether the moisture sensor 1 is situated in the water supply area.

When the sensor state selecting section 73 determines that there is water supply (YES), the sensor state selecting section 73 selects the state C1 (S53). When the sensor state selecting section 73 determines that there is no water supply (NO), the sensor state selecting section 73 selects the state C2 (S54).

As described above, the frequent-communication state (the state C1) is selected when there is expected to be a rapid change in a moisture amount in soil, and the less-frequent-communication state (the state C2) is selected when there is not expected to be a rapid change in a moisture amount in soil. This makes it possible to improve the detection accuracy and to reduce power consumption.

Information regarding the selected sensor state is transmitted to the control apparatus 6 through the communication section 71. The sensor controller 61 of the control apparatus 6 controls the moisture sensor 1 on the basis of the received sensor state information. The processing illustrated in Fig. 10 is performed for each moisture sensor 1.

In the present embodiment, the moisture sensor 1 of which a distance to a portion of the crop 8 that is situated above the ground in the horizontal plane is outside of the threshold is set to be in a communication stop state, as described above. Then, from among the moisture sensors 1 of which the distance to a portion of the crop 8 that is situated above the ground is within the threshold, the moisture sensor 1 situated in an area in which there is water supply is set to be in a frequent-communication state, and the moisture sensor 1 situated in an area in which there is no water supply is set to be in a less-frequent-communication state.

Consequently, a moisture sensor, from among a plurality of moisture sensors, that is situated in an area for which there is estimated to be a need to monitor a moisture amount in soil can be selectively set to be in a communication state. Further, control performed on the frequency in communication performed by the moisture sensor set to be in a communication state results in being able to reduce power consumption of each moisture sensor 1, and thus to reduce power consumption of the entirety of the moisture sensor controlling system 100.

### [Seventh Embodiment]

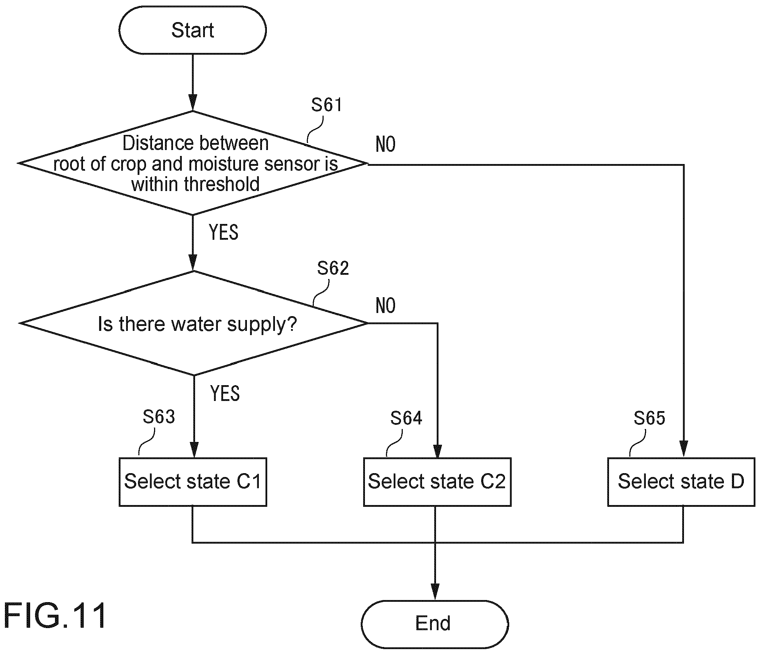

Fig. 11 is a flowchart of the moisture sensor controlling method according to a seventh embodiment. The seventh embodiment is different from the third embodiment only in that options of an operation state of the moisture sensor are different, and a basic flow of the seventh embodiment is similar to that of the third embodiment.

As in the sixth embodiment, an example illustrated in Fig. 11 is an example of controlling an operation state of the moisture sensor 1 by selecting the operation state of the moisture sensor 1 from the state A1, the state A2, and the state B, on the basis of position information regarding a position of the crop 8, water supply information regarding water supply to the soil 80, and position information regarding a position of the moisture sensor 1.

The seventh embodiment is different from the sixth embodiment only in that position information regarding a position of the root 82, of the crop 8, that is situated in the soil 80 is used as the position information regarding a position of the crop 8.

When processing of controlling a moisture sensor is started, the environment data processor 72 of the moisture sensor controlling apparatus 7 acquires, from the control apparatus 6, image data that is acquired by the camera 41. The environment data processor 72 refers to growth information regarding growth of the crop that is stored in the database 74 to estimate a spreading range of the root situated in the soil 80 using the image data, and calculates the position information regarding a position of the root 82. The position information regarding a position of the root 28 of the crop 8 is output to the sensor state selecting section 73.

Further, the environment data processor 72 acquires water-spraying data of water spraying performed by the water spraying apparatus 42 from the control apparatus 6, and acquires rainfall data from the weather server 9. The environment data processor 72 calculates water supply information regarding water supply to the soil 80 using these pieces of information. The calculated water supply information is output to the sensor state selecting section 73.

In the present embodiment, not only position information in the horizontal plane but also position information in the depth direction is considered to control each moisture sensor, as in the third embodiment. This makes it possible to more accurately control a sensor state of each moisture sensor, and thus to reduce power consumption of the entirety of the moisture sensor controlling system 100.

Next, as illustrated in Fig. 11, the sensor state selecting section 73 of the moisture sensor controlling apparatus 7 determines whether a distance between the moisture sensor 1 and the root 82 of the crop 8 is within a threshold, using the position information regarding a position of the root 82 of the crop 8, and using the position information regarding a position of the moisture sensor 1 being stored in the database 74 (S61).

When the sensor state selecting section 73 determines that the distance is within the threshold (YES), the process moves on to S62. When the sensor state selecting section 73 determines that the distance is outside of the threshold (NO), the sensor state selecting section 73 selects the state D (S65).

In S62, on the basis of the water supply information, the sensor state selecting section 73 determines whether there is water supply at a location at which the moisture sensor 1 is situated, that is, whether the moisture sensor 1 is situated in the water supply area.

When the sensor state selecting section 73 determines that there is water supply (YES), the sensor state selecting section 73 selects the state C1 (S63). When the sensor state selecting section 73 determines that there is no water supply (NO), the sensor state selecting section 73 selects the state C2 (S64).

As described above, the frequent-communication state (the state C1) is selected when there is expected to be a rapid change in a moisture amount in soil, and the less-frequent-communication state (the state C2) is selected when there is not expected to be a rapid change in a moisture amount in soil. This makes it possible to improve the detection accuracy and to reduce power consumption.

Information regarding the selected sensor state is transmitted to the control apparatus 6 through the communication section 71. The sensor controller 61 of the control apparatus 6 controls the moisture sensor 1 on the basis of the received sensor state information. The processing illustrated in Fig. 11 is performed for each moisture sensor 1.

In the present embodiment, the moisture sensor 1 of which a distance to a portion of the crop 8 that is situated in the soil 80 is outside of the threshold is set to be in a communication stop state, as described above. Then, from among the moisture sensors 1 of which the distance to the crop 8 is within the threshold, the moisture sensor 1 situated in an area in which there is water supply is set to be in a frequent-communication state, and the moisture sensor 1 situated in an area in which there is no water supply is set to be in a less-frequent-communication state.

Consequently, a moisture sensor, from among a plurality of moisture sensors, that is situated in an area for which there is estimated to be a need to monitor a moisture amount in soil can be selectively set to be in a communication state. Further, control performed on the frequency in communication performed by the moisture sensor set to be in a communication state results in being able to reduce power consumption of each moisture sensor 1, and thus to reduce power consumption of the entirety of the moisture sensor controlling system 100.

### [Eighth Embodiment]

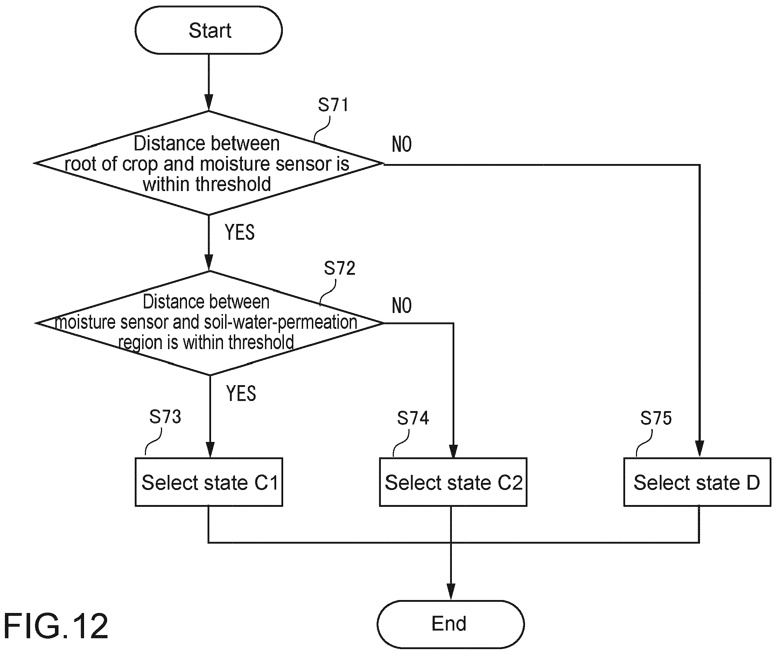

Fig. 12 is a flowchart of the moisture sensor controlling method according to an eighth embodiment. The eighth embodiment is different from the fourth embodiment only in that options of an operation state of the moisture sensor are different, and a basic flow of the eighth embodiment is similar to that of the fourth embodiment.

An example illustrated in Fig. 12 is an example of controlling an operation state of the moisture sensor 1 by selecting the operation state of the moisture sensor 1 from the state C1, the state C2, and the state D, on the basis of position information regarding a position of the root 82 of the crop 8, water supply information regarding water supply to the soil 80, and position information regarding a position of the moisture sensor 1.

In the present embodiment, a soil-water-permeation region (soil-water-depth information) is used as the water supply information regarding water supply to the soil 80. Instead of determining whether there is water supply (S62) in the seventh embodiment, the present embodiment includes determining whether a distance between the soil-water-permeation region and the moisture sensor is within a threshold.

This is described below.

When processing of controlling a moisture sensor is started, the environment data processor 72 of the moisture sensor controlling apparatus 7 acquires, from the control apparatus 6, image data that is acquired by the camera 41, and calculates position information regarding a position of the root 82 of the crop 8 using the image data. The position information regarding a position of the root 82 of the crop 8 is output to the sensor state selecting section 73.

Further, the environment data processor 72 acquires water supply data of water supply performed by the water spraying apparatus 42 from the control apparatus 6, acquires rainfall data from the weather server 9, and acquires soil classification information and soil-water-moving-speed information from the database 74. The environment data processor 72 estimates and calculates a soil-water-permeation region using these pieces of information.

Information regarding the calculated soil-water-permeation region is output to the sensor state selecting section 73.

Next, as illustrated in Fig. 12, the sensor state selecting section 73 of the moisture sensor controlling apparatus 7 determines whether a distance between the moisture sensor 1 and the root 82 of the crop 8 is within a threshold, using the position information regarding a position of the root 82 of the crop 8, and using the position information regarding a position of the moisture sensor 1 being stored in the database 74 (S71) .

When the sensor state selecting section 73 determines that the distance is within the threshold (YES), the process moves on to S72.

When the sensor state selecting section 73 determines that the distance is outside of the threshold (NO), the sensor state selecting section 73 selects the state D (S75).

In S72, the sensor state selecting section 73 determines whether a distance between the soil-water-permeation region and the moisture sensor 1 is within a threshold.

When the sensor state selecting section 73 determines that the distance is within the threshold (YES), the sensor state selecting section 73 selects the state C1 (S73). When the sensor state selecting section 73 determines that the distance is outside of the threshold (NO), the sensor state selecting section 73 selects the state C2 (S74).

Further, on the basis of information regarding an estimated temporal change in the soil-water-permeation region calculated by the environment data processor 72, the sensor state selecting section 73 can control each moisture sensor 1 such as changing an operation state of the moisture sensor 1 to an appropriate state at an appropriate timing.

As described above, control performed on a timing of operating the moisture sensor 1 on the basis of a soil water permeating speed makes it possible to control operation information regarding an operation of a moisture sensor in more detail, and to reduce power consumption efficiently.

Information regarding the selected sensor state is transmitted to the control apparatus 6 through the communication section 71. The sensor controller 61 of the control apparatus 6 controls the moisture sensor 1 on the basis of the received sensor state information. The processing illustrated in Fig. 12 is performed for each moisture sensor 1.

In the present embodiment, the moisture sensor 1 of which a distance to a portion of the crop 8 that is situated in the soil 80 is outside of the threshold is set to be in a transmission stop state, as described above. Further, from among the moisture sensors 1 of which the distance to the portion, of the crop 8, that is situated in the soil 80 is within the threshold, the moisture sensor 1 of which a distance to a soil-water-permeation region is within a threshold is set to be in a frequent-communication state, and the moisture sensor 1 of which the distance to the soil-water-permeation region is outside of the threshold is set to be in a less-frequent-communication state.

Consequently, a moisture sensor, from among a plurality of moisture sensors, that is situated in an area for which there is estimated to be a need to monitor a moisture amount in soil can be selectively set to be in a communication state. Further, control performed on the frequency in communication performed by the moisture sensor set to be in a communication state results in being able to reduce power consumption of each moisture sensor 1, and thus to reduce power consumption of the entirety of the moisture sensor controlling system 100.

The present embodiment makes it possible to optimally control an operation state of the moisture sensor according to the movement of soil water.

### [Other Embodiments]

In the first and fifth embodiments, the examples of respectively selecting whether to perform detection and whether to perform communication to select an operation state have been described. However, how frequent detection is to be performed and how frequent communication is to be performed may be respectively selected. In this case, the state A1 is used instead of the state A, and the state A2 is used instead of the state B in the flowchart of the first embodiment illustrated in Fig. 5. The state C1 is used instead of the state C, and the state C2 is used instead of the state D in the flowchart of the fifth embodiment illustrated in Fig. 9.

Further, the example in which an operation state of the moisture sensor is selected from the operation states A, B, A1, and A2 from the viewpoint of detection, or is selected from the operation states C, D, C1, and C2 from the viewpoint of communication, has been described in the embodiments above. However, the present technology is not limited thereto.

As an example, the operation state of the moisture sensor 1 may be selected from the viewpoint of a frequency band of an electromagnetic wave used upon detecting the moisture sensor 1. For example, in the first embodiment, an ordinary state in which the frequency band of an electromagnetic wave is not changed may be used instead of the state A, and a state in which the frequency band of an electromagnetic wave is changed to be narrower than in the ordinary state may be used instead of the state B. Power consumption can be reduced by narrowing the frequency band. The detection accuracy is reduced by narrowing the frequency band, but the reduction in the detection accuracy is less likely to have an impact since an electromagnetic wave of a narrow frequency band is used for the moisture sensor 1 situated in an area for which there is a relatively low need to monitor a moisture amount in soil. As described above, the operation state of the moisture sensor 1 may be selected from the viewpoint of a frequency band of an electromagnetic wave used upon detection.

Further, the example in which the operation state from the viewpoint of detection and the operation state from the viewpoint of communication are separately used for convenience to select and control the operation state of the moisture sensor has been described in the embodiments above. The operation state of the moisture sensor can also be selected using, in combination, at least two of operation states including the operation state from the viewpoint of detection, the operation state from the viewpoint of communication, and the operation state from the viewpoint of the frequency band of an electromagnetic wave.

Note that control performed on an operation state from each viewpoint can be said to be control performed on an operation state with a different amount of power consumption.

For example, in each of the second to fourth embodiments, frequent communication may be performed in addition to frequent detection being performed in the state A1, and less frequent communication may be performed in addition to less frequent detection being performed in the state A2. As described above, the operation state may be controlled using both of the viewpoints of detection and of communication in combination.

As another example, in each of the second to fourth embodiments, detection using an electromagnetic wave of the frequency band in an ordinary state may be performed in addition to frequent detection being performed in the state A1, and detection using an electromagnetic wave of the frequency band narrower than in the ordinary state may be performed in addition to less frequent detection being performed in the state A2. As described above, the operation state may be controlled using both of the viewpoints of detection and of the frequency band of an electromagnetic wave in combination.

As another example, in each of the second to fourth embodiments, in addition to frequent detection being performed in the state A1, the detection may be performed using an electromagnetic wave of the frequency band in an ordinary state, and frequent communication may be performed. Further, in addition to less frequent detection being performed in the state A2, the detection may be performed using an electromagnetic wave of the frequency band narrower than in the ordinary state, and less frequent communication may be performed. As described above, the operation state may be controlled using the viewpoints of detection, of communication, and of the frequency band of an electromagnetic wave in combination.

As another example, in each of the sixth to eighth embodiments, detection using an electromagnetic wave of the frequency band in an ordinary state may be performed in addition to frequent communication being performed in the state C1, and detection using an electromagnetic wave of the frequency band narrower than in the ordinary state may be performed in addition to less frequent communication being performed in the state C2. As described above, the operation state may be controlled using both of the viewpoints of communication and of the frequency band of an electromagnetic wave in combination.

Further, the example in which the state D is a state in which power is supplied for detection but no transmission power is supplied has been described in each of the fifth to eighth embodiments. As another example, the state D may be a state in which no power is supplied for detection in each of the fifth to eighth embodiments, as in the state B in the first to fourth embodiments. As described above, the operation state may be controlled using both of the viewpoints of communication and of detection in combination.

In all of the examples described above, the moisture sensor can be controlled in an appropriate operation state using environment information regarding an environment of soil and position information regarding a position of the moisture sensor. This makes it possible to reduce power consumption.

As described above, an operation state of the moisture sensor is controlled on the basis of a relationship between environment information regarding an environment of the crop 8 and position information regarding a position of the moisture sensor 1 in each embodiment. This makes it possible to reduce power consumption of each moisture sensor, and thus to reduce power consumption of the entirety of the moisture sensor controlling system 100. This results in being able to reduce costs. When, for example, a battery is used as a power supply, a battery life can be extended, and this results in less frequent battery change. Further, it is possible to pour and spray water with appropriate frequency at an appropriate timing according to the moisture content in soil.

The present technology is particularly effective when a plurality of moisture sensors is installed in soil having a large area, and makes it possible to reduce power consumption of the entirety of a system.

Further, a moisture sensor that achieves a high degree of detection accuracy is desired to be used in order to pour and spray water with more appropriate frequency at a more appropriate timing. For example, the present technology is particularly effective when a moisture sensor that provides a high degree of detection accuracy but consumes a large amount of power is used. The present technology makes it possible to increase the number of options for a used moisture sensor.

For example, it is assumed that the present technology is applied to a mass production system in which a plurality of moisture sensors is installed in soil having a large area in advance and any crops are planted in the soil. This is an example of applying the present technology. Further, a moisture sensor may be installed in soil in which crops are planted in advance.

The embodiments of the present technology have been described above. The present technology is not limited to the embodiments described above, and of course various modifications may be made thereto without departing from the scope of the present technology.

For example, the example in which a plurality of moisture sensors is installed in soil has been described in the embodiments above. However, the present technology can also be applied to a single moisture sensor, and makes it possible to reduce power consumption.

Further, for example, the example in which moisture sensors of a plurality of moisture sensors are intermittently arranged in soil in the horizontal direction and in the depth direction (the vertical direction) has been described in the embodiments above. However, a plurality of moisture sensors may be arranged only in the horizontal direction or only in the depth direction. The present technology can be applied to both of the cases.

Furthermore, the example in which, for example, the sensor state selecting section, the database, and the environment data processor are included in a server (the moisture sensor controlling apparatus) that serves as an information processing apparatus that is different from the moisture amount measuring apparatus and the control apparatus, has been described in the embodiments above. However, the present technology is not limited thereto.

As an example, at least one of the sensor state selecting section, the database, or the environment data processor may be provided to the moisture amount measuring apparatus or control apparatus serving as an information processing apparatus.

Further, as another example, the signal processing unit 5 may be included in the control apparatus 6.

The moisture sensor controlling system 100 may include at least one information processing apparatus, and it is sufficient if the entirety of the system includes a functional structural element such as the sensor state selecting section included in the moisture sensor controlling system 100. This makes it possible to reduce power consumption of the entirety of the moisture sensor controlling system.

Note that the present technology may also take the following configurations.
(1) A moisture sensor controlling apparatus, including
   a sensor state selecting section that controls an operation state of at least one moisture sensor on the basis of environment information regarding an environment of soil in which crops are planted, and on the basis of position information regarding a position of the at least one moisture sensor, the at least one moisture sensor being installed in the soil and detecting a moisture amount in the soil.
(2) The moisture sensor controlling apparatus according to (1), in which
   as the controlling the operation state of the moisture sensor, the sensor state selecting section controls at least one of whether to supply power to the moisture sensor in order to detect the moisture amount in the soil, or how frequent the moisture amount in the soil is to be detected by the moisture sensor.
(3) The moisture sensor controlling apparatus according to (1) or (2), in which
   the moisture sensor includes a communication section that transmits detection data of the detection performed by the moisture sensor, and
   as the controlling the operation state of the moisture sensor, the sensor state selecting section controls at least one of whether the detection data of the detection performed by the moisture sensor is to be transmitted by the communication section, or how frequent the detection data of the detection performed by the moisture sensor is to be transmitted.
(4) The moisture sensor controlling apparatus according to any one of (1) to (3), in which
   the moisture sensor detects relative permittivity of the soil using an electromagnetic wave in order to detect the moisture amount in the soil, and
   as the controlling the operation state of the moisture sensor, the sensor state selecting section controls a band of the electromagnetic wave.
(5) The moisture sensor controlling apparatus according to any one of (1) to (4), in which
   the environment information includes at least one of position information regarding a position of a portion of the crop, or water supply information regarding water supply to the soil.
(6) The moisture sensor controlling apparatus according to (5), further including
   an environment data processor that calculates the environment information using environment data.
(7) The moisture sensor controlling apparatus according to (6), in which
   the environment data processor calculates the position information regarding a position of a portion of the crop on the basis of image data that is the environment data and is acquired by an image-capturing section that captures an image of the crop above the ground, and
   the sensor state selecting section controls the operation state of each moisture sensor using the position information regarding a position of a portion of the crop on the basis of a distance between the portion of the crop and the moisture sensor.
(8) The moisture sensor controlling apparatus according to (7), in which
   the environment data processor calculates, from the image data of the crop, the position information regarding a position of the portion of the crop, the portion of the crop being situated above the ground.
(9) The moisture sensor controlling apparatus according to (7), in which
   the environment data processor
   refers to growth information regarding growth of the crop that is acquired in advance, and
   calculates, from the image data of the crop, the position information regarding a position of the portion of the crop, the portion of the crop being situated in the soil.
(10) The moisture sensor controlling apparatus according to (9), in which
   the moisture sensors of a plurality of the moisture sensors are intermittently arranged in at least one of a horizontal direction of the soil or a depth direction of the soil, and
   the sensor state selecting section controls the operation state of each moisture sensor on the basis of a distance between the position of the portion of the crop and the moisture sensor, the position of the portion of the crop being estimated by the environment data processor, the portion of the crop being situated in the soil.
(11) The moisture sensor controlling apparatus according to any one of (6) to (10), in which
   the environment data processor calculates the water supply information regarding water supply to the soil, using at least one of water supply data of water supply to the soil that is performed by a water spraying apparatus, rainfall data of rainfall in the soil, or detection data of the detection performed by the moisture sensor installed in the soil, the water supply data, the rainfall data, and the detection data being the pieces of environment data.
(12) The moisture sensor controlling apparatus according to any one of (6) to (11), in which
   the water supply information regarding water supply to the soil includes soil-water-permeation-region information regarding a soil-water-permeation region in the soil, and
   the environment data processor calculates the soil-water-permeation-region information using at least one of water supply data of water supply to the soil that is performed by a water spraying apparatus, rainfall data of rainfall in the soil, or detection data of the detection performed by the moisture sensor installed in the soil, and using at least one of soil classification for the soil or a speed at which soil water moves in the soil, the water supply data, the rainfall data, the detection data, the soil classification, and the soil water moving speed being the pieces of environment data.
(13) The moisture sensor controlling apparatus according to any one of (1) to (12), in which
   the sensor state selecting section controls the operation states of the respective moisture sensors of a plurality of the moisture sensors.
(14) A moisture sensor controlling system, including:
   at least one moisture sensor that is installed in soil in which crops are planted, the at least one moisture sensor detecting a moisture amount in the soil; and
   a sensor state selecting section that controls an operation state of the at least one moisture sensor on the basis of environment information regarding an environment of the soil, and on the basis of position information regarding a position of the at least one moisture sensor.
(15) A moisture sensor controlling method, including
   controlling an operation state of at least one moisture sensor on the basis of environment information regarding an environment of soil in which crops are planted, and on the basis of position information regarding a position of the at least one moisture sensor, the at least one moisture sensor being installed in the soil and detecting a moisture amount in the soil.
(16) A moisture sensor that is installed in soil in which crops are planted, the moisture sensor detecting a moisture amount in the soil, in which
   an operation state of the moisture sensor is controlled on the basis of environment information regarding an environment of the soil, and on the basis of position information regarding a position of the moisture sensor.

### Reference Signs List

- 1: moisture sensor
- 7: moisture sensor controlling apparatus
- 8: crop
- 81: leaf (portion, of crop, that is situated above ground)
- 82: root (portion, of crop, that is situated in soil)
- 83: stem (portion, of crop, that is situated above ground)
- 31: communication section
- 41: camera (image-capturing section)
- 42: water spraying apparatus
- 72: environment data processor
- 73: sensor state selecting section
- 80: soil
- 100: moisture sensor controlling system

## Claims

1. A moisture sensor controlling apparatus, comprising
a sensor state selecting section that controls an operation state of at least one moisture sensor on a basis of environment information regarding an environment of soil in which crops are planted, and on a basis of position information regarding a position of the at least one moisture sensor, the at least one moisture sensor being installed in the soil and detecting a moisture amount in the soil.

2. The moisture sensor controlling apparatus according to claim 1, wherein
as the controlling the operation state of the moisture sensor, the sensor state selecting section controls at least one of whether to supply power to the moisture sensor in order to detect the moisture amount in the soil, or how frequent the moisture amount in the soil is to be detected by the moisture sensor.

3. The moisture sensor controlling apparatus according to claim 1, wherein
the moisture sensor includes a communication section that transmits detection data of the detection performed by the moisture sensor, and
as the controlling the operation state of the moisture sensor, the sensor state selecting section controls at least one of whether the detection data of the detection performed by the moisture sensor is to be transmitted by the communication section, or how frequent the detection data of the detection performed by the moisture sensor is to be transmitted.

4. The moisture sensor controlling apparatus according to claim 1, wherein
the moisture sensor detects relative permittivity of the soil using an electromagnetic wave in order to detect the moisture amount in the soil, and
as the controlling the operation state of the moisture sensor, the sensor state selecting section controls a band of the electromagnetic wave.

5. The moisture sensor controlling apparatus according to claim 1, wherein
the environment information includes at least one of position information regarding a position of a portion of the crop, or water supply information regarding water supply to the soil.

6. The moisture sensor controlling apparatus according to claim 5, further comprising
an environment data processor that calculates the environment information using environment data.

7. The moisture sensor controlling apparatus according to claim 6, wherein
the environment data processor calculates the position information regarding a position of a portion of the crop on a basis of image data that is the environment data and is acquired by an image-capturing section that captures an image of the crop above the ground, and
the sensor state selecting section controls the operation state of each moisture sensor using the position information regarding a position of a portion of the crop on a basis of a distance between the portion of the crop and the moisture sensor.

8. The moisture sensor controlling apparatus according to claim 7, wherein
the environment data processor calculates, from the image data of the crop, the position information regarding a position of the portion of the crop, the portion of the crop being situated above the ground.

9. The moisture sensor controlling apparatus according to claim 7, wherein
the environment data processor
refers to growth information regarding growth of the crop that is acquired in advance, and
calculates, from the image data of the crop, the position information regarding a position of the portion of the crop, the portion of the crop being situated in the soil.

10. The moisture sensor controlling apparatus according to claim 9, wherein
the moisture sensors of a plurality of the moisture sensors are intermittently arranged in at least one of a horizontal direction of the soil or a depth direction of the soil, and
the sensor state selecting section controls the operation state of each moisture sensor on a basis of a distance between the position of the portion of the crop and the moisture sensor, the position of the portion of the crop being estimated by the environment data processor, the portion of the crop being situated in the soil.

11. The moisture sensor controlling apparatus according to claim 6, wherein
the environment data processor calculates the water supply information regarding water supply to the soil, using at least one of water supply data of water supply to the soil that is performed by a water spraying apparatus, rainfall data of rainfall in the soil, or detection data of the detection performed by the moisture sensor installed in the soil, the water supply data, the rainfall data, and the detection data being the pieces of environment data.

12. The moisture sensor controlling apparatus according to claim 6, wherein
the water supply information regarding water supply to the soil includes soil-water-permeation-region information regarding a soil-water-permeation region in the soil, and
the environment data processor calculates the soil-water-permeation-region information using at least one of water supply data of water supply to the soil that is performed by a water spraying apparatus, rainfall data of rainfall in the soil, or detection data of the detection performed by the moisture sensor installed in the soil, and using at least one of soil classification for the soil or a speed at which soil water moves in the soil, the water supply data, the rainfall data, the detection data, the soil classification, and the soil water moving speed being the pieces of environment data.

13. The moisture sensor controlling apparatus according to claim 1, wherein
the sensor state selecting section controls the operation states of the respective moisture sensors of a plurality of the moisture sensors.

14. A moisture sensor controlling system, comprising:
at least one moisture sensor that is installed in soil in which crops are planted, the at least one moisture sensor detecting a moisture amount in the soil; and
a sensor state selecting section that controls an operation state of the at least one moisture sensor on a basis of environment information regarding an environment of the soil, and on a basis of position information regarding a position of the at least one moisture sensor.

15. A moisture sensor controlling method, comprising
controlling an operation state of at least one moisture sensor on a basis of environment information regarding an environment of soil in which crops are planted, and on a basis of position information regarding a position of the at least one moisture sensor, the at least one moisture sensor being installed in the soil and detecting a moisture amount in the soil.
